# FASCICULE DE BREVET EUROPEEN

(11) **EP 2 018 396 B1**
(45) Date de publication et mention de la délivrance du brevet: **06.10.2010**
(21) Numéro de dépôt: 07731404.5
(22) Date de dépôt: 03.05.2007
(51) Int. Cl.: C07K 14/395, G01N 33/50

(54) **MODELISATION CHEZ LA LEVURE DES MUTATIONS DU GENE MITOCHONDRIAL ATP6 RESPONSABLES DU SYNDROME NARP CHEZ L'HOMME ET SES APPLICATIONS POUR LE CRIBLAGE DE MEDICAMENTS**
MODELLIEREN MIT HEFE BEI MITOCHONDRIALEN ATP6-GEN MUTATIONEN VERANTWORTLICH FÜR DAS DAS NARP-SYNDROM BEIM MENSCHEN, UND DIE ANWENDUNGEN DAVON ZUM SCREENING VON MEDIKAMENTEN
MODELLING IN YEAST OF THE MITOCHONDRIAL ATP6 GENE MUTATIONS RESPONSIBLE FOR NARP SYNDROME IN HUMANS AND USES THEREOF FOR SCREENING FOR MEDICAMENTS

(30) Priorité: 03.05.2006 FR 0603934
(43) Date de publication de la demande: 28.01.2009
(73) Titulaire: Université Victor Segalen Bordeaux 2, 33076 Bordeaux Cedex (FR); Centre National de la Recherche Scientifique, 75016 Paris (FR)
(72) Inventeur: DI RAGO, Jean-Paul, 33480 Sainte-Hélène (FR); RAK, Malgorzata, 26-060 Checiny (PL); KUCHARCZYK, Roza, 01-355 Warszawa (PL); TETAUD, Emmanuel, 33600 Pessac (FR); DUVEZIN-CAUBET, Stéphane, 40350 Mimbaste (FR)
(74) Mandataire: Leblois-Préhaud, Hélène Marthe Georgette
(86) Numéro de dépôt international: PCT/FR2007/000757
(87) Numéro de publication internationale: WO 2007/125225

(56) Documents cités:
- BONGORNO ET AL.: "Developing a Mouse Model of the Mitochondrial NARP Syndrome Using AAV-Ribozymes for ATP6" MOLECULAR THERAPY, ACADEMIC PRESS, SAN DIEGO, CA,, US, vol. 9, mai 2004 (2004-05), page 167, XP004634803 ISSN: 1525-0016
- SCHON ERIC A ET AL: "Pathogenesis of primary defects in mitochondrial ATP synthesis" SEMINARS IN CELL AND DEVELOPMENTAL BIOLOGY, vol. 12, no. 6, décembre 2001 (2001-12), pages 441-448, XP002403535 ISSN: 1084-9521 cité dans la demande
- HOLT I J ET AL: "A NEW MITOCHONDRIAL DISEASE ASSOCIATED WITH MITOCHONDRIAL DNA HETEROPLASMY" AMERICAN JOURNAL OF HUMAN GENETICS, vol. 46, no. 3, 1990, pages 428-433, XP009073979 ISSN: 0002-9297
- CELOTTO ALICIA M ET AL: "Mitochondrial encephalomyopathy in Drosophila" JOURNAL OF NEUROSCIENCE, vol. 26, no. 3, janvier 2006 (2006-01), pages 810-820, XP002403537 ISSN: 0270-6474
- JOHN U P ET AL: "SEQUENCE OF THE MITOCHONDRIAL OLI2 GENE CODING FOR SUBUNIT 6 OF THEMITOCHONDRIAL ATPASE COMPLEX IN DIFFERENT STRAINS OF SACCHAROMYCES" NUCLEIC ACIDS RESEARCH, OXFORD UNIVERSITY PRESS, SURREY, GB, vol. 15, no. 1, 1987, page 366, XP000971250 ISSN: 0305-1048
- GUELIN E ET AL: "Isolation of the ATP synthase subunit 6 and sequence of the mitochondrial ATP6 gene of the yeast Candida parapsilosis" EUROPEAN JOURNAL OF BIOCHEMISTRY 1991 GERMANY, vol. 197, no. 1, 1991, pages 105-111, XP002403538 ISSN: 0014-2956

## Description

La présente invention est relative à des souches de levure portant l'équivalent de mutations du gène mitochondrial *ATP6* responsables du syndrome NARP chez l'homme et leurs applications pour le criblage de médicaments actifs contre les pathologies mitochondriales impliquant un déficit de production d'ATP par la voie de la phosphorylation oxydative comme le syndrome NARP.

Le NARP -(Neuropathie, Ataxie et Rétinite Pigmentaire) est un syndrome héréditaire transmis maternellement et caractérisé par un retard de développement, accompagné de rétinite pigmentaire (RP), démence, ataxie, faiblesse musculaire neurologique proximale et de neuropathies sensorielles (Schon et al., J. Bioenerg. Biomembr., 1994, 26, 291-299 ; Graeber, M.B. et Müller, U., J. Neurol-Sci., 1998, 153, 251-263, pour revue). Cette maladie est d'une manière générale une pathologie de l'enfant mais elle a également été rapportée dans des cas plus rares, chez l'adulte. Les manifestations cliniques sont variées et peuvent prendre des formes plus ou moins sévères. Ainsi, les manifestations ophtalmiques peuvent aller d'un simple changement dit 'poivre et sel' de la rétine à une RP sévère, accompagnée de maculopathie. De même, les manifestations neurologiques sont comprises dans un large spectre qui va de simples migraines à des démences sévères et à la maladie de Leigh (Encéphalopathie sub-aigüe nécrosante ou *Subacute necrotising encephalomyelopathy* ; Ortiz et al., Arch., Ophtalmol., 1993, 111, 1525-1530). Il existe de nombreux syndromes associés à la rétinite pigmentaire tels que le syndrome d'Usher dans lequel à la fois la vision et l'ouïe sont affectées, ou encore la dystrophie maculaire, aussi appelée RP inversée.

En 1990, Holt et al. (Am. J. Hum. Genet., 46, 428-433) ont décrit pour la première fois la présence de la mutation t8993g (ou T8993G) dans l'ADN mitochondrial de patients présentant le syndrome NARP/maladie de Leigh. Il a alors ensuite été postulé par Tatuch et Robinson (Biochem. Biophys. Res. Commun., 1993, 192, 124-128) que cette mutation conduisait à la réduction de la synthèse d'ATP en altérant le complexe ATP synthase mitochondrial. Cette mutation serait responsable d'un défaut d'assemblage/stabilité de l'ATP synthase (Nijtmans et al., J. Biol. Chem., 2001, 276, 6755-6762). D'autres mutations du gène *ATP6* ont également été détectées, en association avec le syndrome NARP/maladie de Leigh : t8993c, t9176g, t9176c et t8851c (Schon et al., Cell & Dev. Biol., 2001, 12, 441-448). Une simple mutation ponctuelle est donc responsable de ce syndrome qui revêt de nombreuses formes plus ou moins graves. La grande diversité des manifestations pathologiques est attribuée au caractère hétéroplasmique de cette mutation chez les patients, c'est-à-dire la coexistence dans les cellules ou tissus de molécules d'ADN mitochondrial mutées et sauvages. La charge en ADN mitochondrial muté est en étroite corrélation avec la gravité des symptômes observés (Uziel et al, J. Neurol. Neurosurg. Psychiatry, 1997, 63, 16-22 ; Carelli et al, Arch. Neurol., 2002, 59, 264-270). Par exemple, l'encéphalopathie de Leigh est observée lorsque la proportion d'ADN mitochondrial muté est très élevée (>90-95%). Lorsque la mutation est présente dans une proportion plus faible (<75%), elle conduit au développement du syndrome NARP (Shoffner et al., Neurology, 1992, 42, 2168-2174 ; Ortiz et al., Arch., Ophtalmol., 1993, 111, 1525-1530 ; Wallace DC, Science, 1999, 283, 1482-1488, pour revue ; Graeber, M.B. et Mûller, U., J. Neurol. Sci., 1998, 153, 251-263, pour revue).

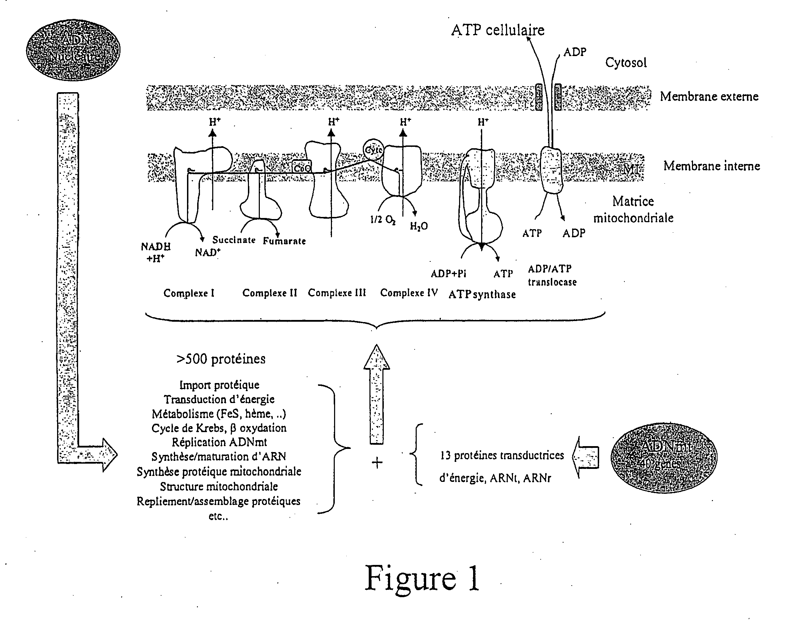

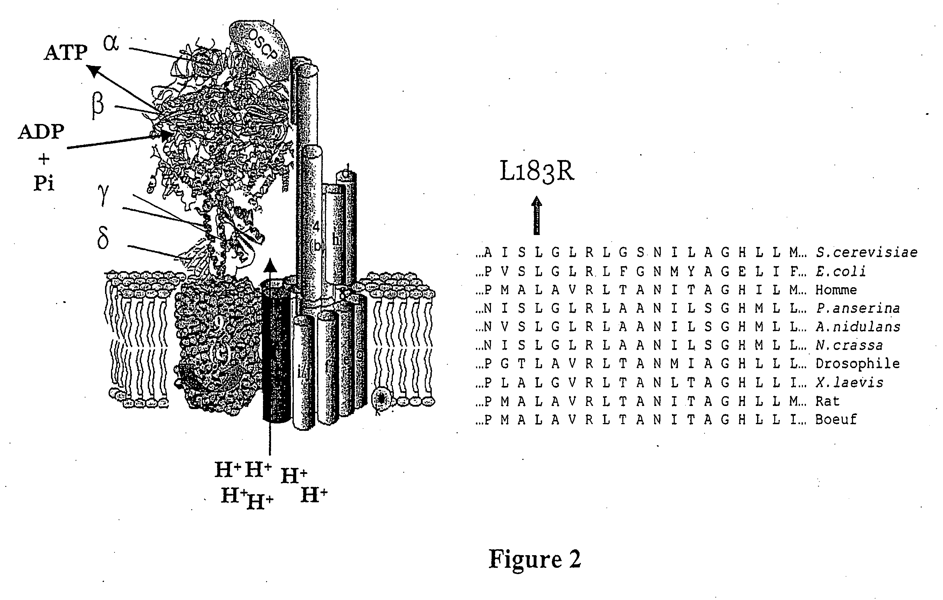

Le complexe ATP synthase, qui est la cible de la mutation t8993g, est localisé dans la membrane mitochondriale interne (Figures 1 et 2). Il catalyse les dernières étapes de la phosphorylation oxydative, un processus qui permet aux cellules d'extraire l'énergie chimique des métabolites et de stocker cette énergie dans des molécules d'ATP. Pour synthétiser l'ATP, le complexe ATP synthase utilise le gradient électrochimique de protons de part et d'autre de la membrane interne, généré par d'autres complexes localisés dans cette membrane, les complexes respiratoires (Figure 1). Ceux-ci transfèrent à l'oxygène les équivalents réducteurs des substrats oxydés dans la mitochondrie. Ces transferts sont couplés à des transports de protons (ions hydrogène, H⁺) à travers la membrane interne, de l'intérieur (la matrice mitochondriale) dans l'espace compris entre les membranes externe et interne (espace intermembranaire) de l'organelle. Le résultat est une concentration en protons plus forte à la périphérie externe de la membrane interne qu'à sa périphérie interne. Le domaine membranaire (Fₒ) de l'ATP synthase permet un retour canalisé des protons dans la matrice mitochondriale. Ce transport est couplé à une synthèse d'ATP dans le domaine catalytique Fᵢ de l'ATP synthase localisé à l'extérieur de la membrane, dans la matrice mitochondriale. L'ATP synthase fonctionne comme une turbine rotative : le passage des protons dans le Fₒ est couplé à la rotation d'un sous-complexe (le rotor) de l'enzyme. Cette rotation conduit à des changements de conformation dans le Fᵢ qui favorisent la synthèse de l'ATP à partir d'ADP et de phosphate inorganique (Boyer P.D., Annu. Rev., Biochem., 1997, 66, 717-747). Les molécules d'ATP néosynthétisées peuvent, *via* un transporteur spécifique localisé dans la membrane interne (l'ADP/ATP translocase), quitter le compartiment mitochondrial pour fournir en énergie l'ensemble de la cellule. L'ATP synthase comprend une vingtaine de sous-unités protéiques différentes pour une masse d'environ 600 KDa. Chez l'homme, deux sous-unités de l'ATP synthase (Atp6p et Atp8p) sont codées par le génome mitochondrial, toutes les autres sous-unités étant codées par des gènes nucléaires. Les sous-unités d'origine nucléaire sont synthétisées dans le cytosol puis importées dans la mitochondrie, tandis que les sous-unités Atp6p et Atp8p codées par le génome mitochondrial sont synthétisées à l'intérieur même de la mitochondrie.

La mutation t8993g associée au syndrome NARP est localisée dans le gène mitochondrial *ATP6.* Celui-ci code pour la sous-unité 6 de l'ATP synthase (Atp6p) essentielle pour le transport des protons à travers le Fₒ (Figure 2). La mutation t8993g conduit au remplacement par l'arginine d'un résidu leucine conservé dans toutes les séquences connues d'Atp6p, des bactéries à l'homme. Ce résidu leucine est dans une région d'Atp6p présumée transmembranaire et critique pour l'activité de translocation des protons de l'ATP synthase. Des études menées chez la bactérie *Escherischia coli* ou avec des cybrides NARP (cellules humaines dont les mitochondries sont enrichies, jusqu'à 100%, en allèles t8993g) indiquent que la mutation t8993g affecte bien le fonctionnement du canal à protons de l'ATP synthase et que ce défaut est la cause primaire de la maladie (Schon et al., Cell & Dev. Biol., 2001, 12, 441-448 ; Nijtmans et al., J. Biol. Chem., 2001, 276, 6755-6762).

Il n'existe pas actuellement de médicament efficace pour le traitement du syndrome NARP, ni de modèle cellulaire de ce syndrome adapté au criblage massif de molécules d'intérêt thérapeutique.

En effet, l'utilisation de cybrides dérivées de cellules humaines implique une étape de culture cellulaire qui est longue, délicate, laborieuse et onéreuse pour les cellules de mammifères qui se divisent lentement (temps de doublement d'au moins 24 heures), requièrent des milieux de culture complexes, sont sensibles aux contamination par des microorganismes et ne peuvent pas être cultivées sur un milieu solide (gélosé). En outre, les cybrides sont des cellules peu sensibles pour l'étude du syndrome NARP.

D'autre part, les bactéries n'ont pas de mitochondries, et à ce titre elles ne sont pas un bon modèle pour étudier l'impact des mutations pathogènes du gène *ATP6* sur la physiologie mitochondriale. En outre, même si les ATP synthases de bactéries et de cellules eukaryotes (mammifères, levure) fonctionnent de manière semblable, elles possèdent cependant des différences de structure importante avec notamment une dizaine de sous-unités additionnelles ou 'surnuméraires' dans les ATP synthases eucaryotes qui n'ont pas d'équivalent chez les bactéries (Velours, J. et Arselin, G., J. Bioenerg. Biomembr., 2000, 32, 383-390).

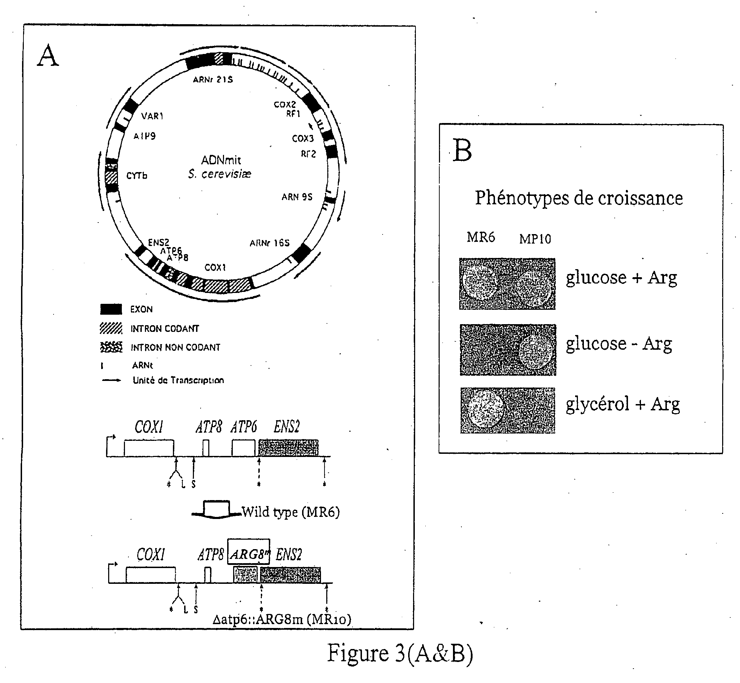

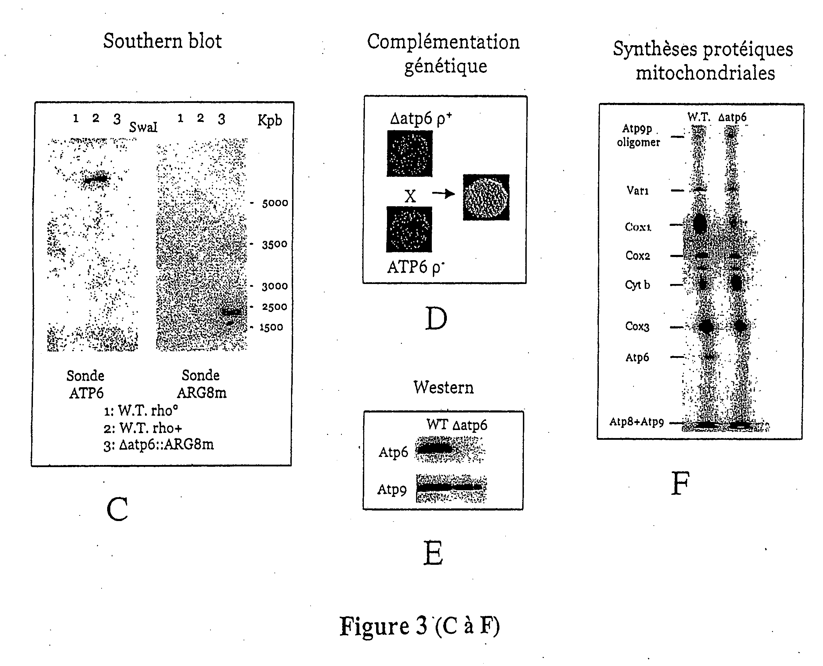

La levure de boulangerie *Saccharomyces cerevisiae,* un champignon unicellulaire, est depuis plusieurs dizaines d'années un modèle de référence pour l'étude des mitochondries. Un avantage décisif est la bonne capacité fermentaire de la levure, de sorte que cet organisme est capable de survivre à des mutations qui inactivent le systèmé énergétique mitochondrial. On arrive ainsi à maintenir en vie correctement des mutants qui ne synthétisent plus l'ATP par la voie mitochondriale. En particulier, la levure est un bon modèle pour l'isolement et l'étude de mutants de l'ADN mitochondrial. Le génome mitochondrial de la levure, comme chez l'homme, est une petite molécule d'ADN double-brin circulaire. Chez la levure, ce génome code (Figure 3) pour sept sous-unités du système énergétique mitochondrial : une sous-unité du complexe III (cytochrome b), trois sous-unités du complexe IV (Cox1p, Cox2p, Cox3p) et trois sous-unités de l'ATP synthase (Atp6p, Atp8p et Atp9p). Il contient également quelques gènes nécessaires au système de synthèse protéique mitochondrial : une sous-unité protéique (Var1) et les composantes ARN (15S et 21S) du mitoribosome, et un jeu de 24 ARN de transfert suffisant au décryptage de toutes les phases ouvertes de lecture du génome mitochondrial. Le génome mitochondrial est donc uniquement requis pour l'expression de la phosphorylation oxydative. C'est la raison pour laquelle la levure grâce à sa bonne capacité fermentaire est capable de survivre à la perte des gènes mitochondriaux.

Le génome mitochondrial est présent en plusieurs copies par cellule, des milliers dans les cellules humaines, une cinquantaine chez la levure. Toutefois, alors que les mutations du génome mitochondrial, comme la mutation t8993g, sont hétéroplasmiques dans les cellules de mammifères, chez la levure, l'hétéroplasmie est normalement instable. De ce fait, chez la levure, il est possible d'obtenir des clones purs (homoplasmiques) où toutes les molécules d'ADN mitochondrial portent une mutation donnée. Cela permet d'analyser avec précision les effets d'une mutation donnée de l'ADN mitochondrial. La levure est l'un des rares organismes chez lesquels il est possible, par un procédé biolistique, d'introduire des mutations définies dans le génome mitochondrial (Bonnefoy, N et Fox, T.D., Methods Cell. Biol., 2001, 65, 381-396).

La mutagenèse dirigée de l'ADN mitochondrial est une technique bien maîtrisée chez la levure, et de nombreuses mutations ont déjà été introduites avec succès dans cet ADN, notamment dans les gènes *COB-BOX* et *COX2* (Bonnefoy et al., Mol. Cell. Biol., 2001, 21, 2359-2372). Toutefois, jusqu'à présent aucune mutation définie n'a pu être introduite avec succès dans les gènes mitochondriaux de l'ATP synthase.

En effet, dans les années soixante dix, des mutations ponctuelles délétères de l'ADN mitochondrial (*mit*⁻) ont été recherchées en vue de mieux définir les gènes mitochondriaux et les lois qui régissent leur transmission (Slonimski, P.P. et Tzagoloff, A., Eur., J. Biochem., 1976, 61, 27-41). Les mutants avaient été isolés dans un contexte nucléaire *op1*. Dans ce contexte la mutation de perte du génome mitochondrial (ρ⁻/ρ°) est létale et donc non sélectionnable. L'avantage est de faciliter l'obtention de mutations *mit* car ceux-ci apparaissent à des fréquences beaucoup plus faibles (10⁻⁵ à 10⁻⁸) que la mutation ρ⁻/ρ° (10⁻²). Par cette approche, des centaines de mutants *mit*⁻ ont été isolés et caractérisés. Toutefois, aucun des mutants obtenus n'affectait l'un des gènes mitochondriaux de l'ATP synthase (*ATP6, ATP8* et *ATP9*)*.* De ces études, a été tiré l'enseignement selon lequel des mutations *mit*⁻ dans ces gènes sont incompatibles avec le maintien du génome mitochondrial. Cet enseignement a été renforcé ultérieurement par le fait que des mutations nucléaires de l'ATP synthase (notamment dans les gènes *ATP16* et *ATP3*) déstabilisent massivement le génome mitochondrial avec une accumulation exclusive en cellules ρ⁻/ρ° (Velours, J. et Arselin, G., J. Bioenerg. Biomembr., 2000, 32, 383-390).

Contrairement à cet enseignement bien établi, fondé sur des études antérieures, les inventeurs ont montré qu'il était possible d'obtenir des mutations *mit*⁻, sous une forme stable, dans les gènes mitochondriaux de l'ATP synthase.

En effet, les inventeurs ont mis au point un système facilitant l'obtention de mutations du gène *ATP6.* A l'aide de ce système, ils ont réussi, pour la première fois, la construction de souches de levure portant l'équivalent de mutations du gène mitochondrial *ATP6* responsables du syndrome NARP chez l'homme. Les analyses ont montré que ces mutations altèrent plus ou moins sévèrement le fonctionnement de l'ATP synthase de levure, comme chez l'homme ; trois de ces mutants présentent un retard très important de croissance à partir d'une source de carbone non fermentescible (comme le glycérol). En revanche, ces mutants croissent normalement en présence de glucose, un substrat qui permet chez la levure une production efficace d'ATP par la fermentation, qui ne requière pas la présence d'un complexe ATP synthase fonctionnel.

De ce fait, ces mutants de levure, peuvent avantageusement être utilisés pour la recherche de médicaments capables d'atténuer les effets délétères causés par les mutations associées au syndrome NARP, notamment par criblage de chimiothèques. Ces mutants de levure permettent d'identifier des molécules capables de corriger les effets de la mutation en restaurant, soit le fonctionnement de l'ATP synthase, soit une production suffisante d'ATP dans les mitochondries, par une autre voie que celle de la phosphorylation oxydative. Les molécules capables de restaurer le fonctionnement de l'ATP synthase sont potentiellement utilisables comme médicament pour le traitement du syndrome NARP. Les molécules capables de restaurer la production d'ATP dans les mitochondries sont potentiellement utilisables comme médicament pour le traitement non seulement du syndrome NARP, mais aussi d'autres pathologies mitochondriales impliquant un déficit de production d'ATP par la voie de la phosphorylation oxydative ; il s'agit notamment de pathologies liées à un dysfonctionnement des complexes respiratoires telles que les syndromes LHON *(Leber's Hereditary Ootic Neuropathy*), MILS (*Maternally Inherited Leigh Syndrome*)*,* MERRF (*Myoclonic Epilepsy with Ragged-Red Fibers*) et HSP (*Hereditary Spastic Paraplegia*)*.*

Les drogues sont sélectionnées pour leur capacité à restaurer la croissance respiratoire du mutant de levure. La technologie de criblage avec des cellules de levure est bien maîtrisée et a déjà été utilisée avec succès pour identifier des molécules anti-prion actives tant dans les modèles levures que mammifères (Bach et al., Nature Biotechnology, 2003, 21, 1075-1081). Un tel criblage avec la levure est simple à mettre en oeuvre, rapide, peu coûteux, facilement automatisable, et des dizaines de milliers de molécules peuvent ainsi être testées en quelques mois à peine.

En conséquence, la présente invention a pour objet une cellule de levure modifiée, caractérisée en ce que qu'elle comprend une mutation du codon tryptophane 136 (W₁₃₆), leucine 183 (L₁₈₃) ou leucine 247 (L₁₄₇) du gène mitochondrial *ATP6.*

### Définitions

- les termes «cellule de levure», «souche de levure», «cellule», « levure » et « souche » sont considérés comme équivalents dans le contexte de la présente invention et employés indifféremment ; il en va de même pour les souches de levure *rho⁺, rho*⁰ et *rho*⁻ telles que définies ci-après.
- Souche *rho⁺* + (ρ+:) souche de levure comprenant un ADN mitochondrial intact et fonctionnel comme dans des souches sauvages.
- Souche *rho*⁰ (ρ⁰): souche de levure dépourvue d'ADN mitochondrial, caractérisée par une incapacité de croissance dans un milieu contenant une source de carbone non fermentescible et une absence de synthèse protéique mitochondriale.
- Souche *rho⁻* (ρ⁻) synthétique : souche de levure initialement *rho⁰* dont les mitochondries ont été transformées par un ADN exogène (ADN hétérologue), notamment par la méthode de bombardement biolistique de cellules de levure précitée. Cette transformation est rendue possible par la capacité des cellules de levure de pouvoir répliquer et maintenir n'importe quel fragment d'ADN, notamment un vecteur bactérien (plasmide). Comme dans une souche *rho⁻* naturelle, l'ADN introduit artificiellement dans les mitochondries sera réitéré de manière à produire dans l'organelle une masse d'ADN équivalente à celle présente dans des mitochondries de souche sauvage *rho*⁺. Le gène d'intérêt sera donc présent dans la souche *rho⁻* synthétique en un nombre relativement élevé de copies (plus de 3000 pour un gène d'intérêt d'environ 1kb). C'est donc pour ces propriétés proches de celles des cellules *rho⁻* (naturelles) que de telles cellules sont appelées par analogie cellules *rho*⁻ synthétiques.
- Souche mit⁻ : souche de levure comprenant une altération locale (substitution nucléotidique, courte délétion ou insertion) dans la séquence d'un gène mitochondrial qui code pour une des sous-unités du système énergétique mitochondrial.
- Transformants mitochondriaux : transformants obtenus notamment par bombardement des cellules de levure, selon la méthode biolistique précitée. Le bombardement d'une souche *rho⁰* conduit à l'obtention de transformants mitochondriaux qui sont des *rho⁻* synthétiques. Tout vecteur peut être utilisé pour les bombardements, mais pour identifier les transformants mitochondriaux, il faut un vecteur comprenant un marqueur du génome mitochondrial de la levure, par exemple le gène *COX2,* ou un fragment dudit gène.
- Recombinants mitochondriaux : ils sont obtenus par recombinaison homologue après mise en présence de la souche *rho⁻* synthétique avec une souche *rho⁺.*
- Banque de molécules ou chimiothèque : un ensemble de molécules, apparentées par leur structure, leur origine ou leur fonction, notamment une banque combinatoire incluant des molécules qui diffèrent entre elles par le remplacement systématique ou aléatoire de leurs constituants élémentaires, par exemple une banque d'oligomères tels que des peptides, des oligonucléotides (aptamères) et des oligosaccharides ou bien une banque de molécules organiques autres que des oligomères, cycliques ou non-cycliques, notamment des petites molécules organiques, c'est-à-dire de masse moléculaire inférieure à 2500 Da, de préférence inférieure à 2000 Da, de manière préférée inférieure à 1500 Da, de manière plus préférée inférieure à 1000 Da, de manière encore plus préférée inférieure à 750 Da.
- gène *ATP6 :* le gène correspondant aux positions 28487 à 29266 dans le génome mitochondrial de *Saccharomyces cerevisiae* (numéro d'accès NCBI NC_001224.1) ou à la séquence SEQ ID NO : 1 dans la liste de séquences jointe en annexe. Le codon 136 (positions 406-408 de la séquence nucléotidique SEQ ID NO : 1) est tga qui spécifie un résidu tryptophane (W). Le codon 183 (positions 547-549 de la séquence nucléotidique SEQ ID NO : 1) est tta qui spécifie un résidu leucine (L). Le codon 247 (positions 739-741 de la séquence nucléotidique SEQ ID NO : 1) est tta qui spécifie un résidu leucine (L).
- Atp6p : la protéine codée par le gène *ATP6.* La séquence d'Atp6p de la levure *S. cerevisiae* présente le numéro d'accès Swiss-Prot P00854 et correspond à la séquence SEQ ID NO : 2 dans la liste de séquences jointe en annexe ; l'acide aminé en position 136 est un résidu tryptophane (W), et les acides aminés en positions 183 et 247 sont des résidus leucine (L).
- mutation d'un codon : la substitution ou la délétion d'un ou plusieurs nucléotides d'un codon, ainsi que l'insertion d'une séquence nucléotidique dans un codon.

Conformément à l'invention, la mutation du gène *ATP6* est une mutation délétère, c'est-à-dire une mutation qui altère l'activité de l'ATP synthase Atp6p. Cette altération peut être évaluée, *in vitro* ou *in vivo,* par toute technique connue-de l'homme du métier. Parmi les techniques, *in vitro* (à partir de mitochondries isolées), on peut citer notamment : la mesure de la synthèse d'ATP par le complexe ATP synthase, l'analyse du potentiel électrique mitochondrial, et la mesure de la vitesse de consommation d'oxygène des mitochondries, en présence de NADH comme substrat respiratoire. Parmi les techniques, *in vivo,* on peut citer notamment, l'analyse de la croissance respiratoire des levures, c'est-à-dire en présence d'une source de carbone non-fermentescible.

Selon un mode de réalisation avantageux de ladite cellule, la dite mutation est une substitution du codon tryptophane ou leucine par un codon arginine ou proline, de préférence un codon arginine, de manière préférée un codon aga.

De manière préférée, ladite mutation est sélectionnée dans le groupe constitué par : W 136R, L183R et L247R.

Selon un autre mode de réalisation avantageux de ladite cellule, elle dérive d'une souche *rho⁺* de *Saccharomyces cerevisiae,* telle que notamment la souche W303-1B (*MATα, leu2-3, leu2-112, trp1-1, ura3-1, his3-11, his3-15, ade2-1, can1-100 ;* ATCC # 201238).

La souche de levure selon l'invention peut être produite par croisement d'un transformant mitochondrial (souche *rho*⁻ synthétique) ne contenant que le gène *ATP6* muté dans ses mitochondries, avec une souche sauvage (souche *rho⁺,* contenant un génome mitochondrial sauvage incluant le gène *ATP6* sauvage), et isolement des recombinants haploïdes (cytoductants) contenant un génome mitochondrial muté, incluant la mutation du codon 136, 183 ou 247 du gène *ATP6.*

Alternativement, la souche de levure peut être produite en deux étapes, pour faciliter l'isolement des mutants. Dans une première étape, le gène mitochondrial *ATP6* est délété et remplacé par un marqueur génétique indépendant de la fonction respiratoire tel que notamment *ARG8^{m};* cette étape est effectuée par croisement d'un transformant mitochondrial (souche *rho⁻* synthétique) contenant une cassette d'inactivation du gène *ATP6* dans ses mitochondries, avec une souche sauvage, et isolement des recombinants haploïdes (cytoductants) contenant un génome mitochondrial muté dans lequel le gène *ATP6* est remplacé par le marqueur génétique. Dans une seconde étape, le recombinant obtenu à la première étape est croisé avec un transformant mitochondrial (souche *rho⁻* synthétique) ne contenant que le gène *ATP6* muté dans ses mitochondries. Les recombinants haploïdes (cytoductants) contenant un génome mitochondrial muté dans lequel le marqueur génétique est remplacé par un *gène ATP6* muté au niveau du codon 136, 183 ou 247.

Les acides nucléiques sont manipulés selon les méthodes classiques de biologie moléculaire, à l'aide des protocoles standards tels que décrits dans: Current Protocols in Molecular Biology (Frederick M. A USUBEL, 2000, Wiley and son Inc, Library of Congress, USA) et Molecular Cloning: A Laboratory Manual, Third Edition, (Sambrook et al, 2001, Cold Spring Harbor, New York: Cold Spring Harbor Laboratory Press).

La transformation des mitochondries et les manipulations du génome mitochondrial sont effectuées selon les techniques classiques telles que décrites dans Bonnefoy N et Fox T.D., Mol. Gen. Genet., 2000, 262, 1036-1046 et Methods Cell. Biol., 2001, 65, 381-396.

La présente invention a également pour objet l'utilisation d'une cellule de levure modifiée telle que définie ci-dessus, pour le criblage de médicaments actifs contre les pathologies mitochondriales impliquant un déficit de production d'ATP par la voie de la phosphorylation oxydative.

Ces pathologies sont liées à un dysfonctionnement du système énergétique mitochondrial, telles que notamment le syndrome NARP, lié à un dysfonctionnement de l'ATP synthase et les syndromes LHON (*Leber's Hereditary Ootic Neuropathy*)*,* MILS (*Maternally Inherited Leigh Syndrome*)*,* MERRF *(Myoclonic Epilepsy with Ragged-Red Fibers)* et HSP (Hereditary Spastic Paraplegia), liés à un dysfonctionnement des complexes respiratoires.

La présente invention a également pour objet un procédé de criblage de médicaments actifs contre les pathologies mitochondriales impliquant un déficit de production d'ATP par la voie de la phosphorylation oxydative, caractérisé en ce qu'il comprend:
a) la culture d'une cellule de levure modifiée, telle que définie ci-dessus, en présence d'une molécule à tester, dans un milieu contenant une source de carbone non-fermentescible, et
b) l'identification des molécules capables de restaurer la croissance desdites cellules de levure modifiées.

Chez les souches mutantes de levure, en particulier les mutants L183R, L247R et W136R, le dysfonctionnement de l'ATP synthase se traduit par un retard très important de croissance à partir d'une source de carbone non fermentescible comme le glycérol. Par conséquent, ces mutants de levure permettent d'identifier des molécules capables d'atténuer les effets délétères causés par la mutation ; le criblage est effectué sur la restauration de la croissance du mutant dans un milieu de culture contenant une source de carbone non-fermentescible.

Conformément à l'invention, le milieu de culture est liquide ou solide. De préférence, il s'agit d'un milieu solide, tel qu'un milieu gélosé. Lorsque le milieu de culture est liquide, la molécule à tester est ajoutée dans le milieu. Lorsque le milieu de culture est solide, les levures sont ensemencées à la surface du milieu et la molécule à tester est mise en contact avec la levure, notamment par application de la molécule à tester sur les levures, par exemple à l'aide d'un filtre (membrane poreuse) contenant la molécule à tester. La culture est réalisée dans des conditions permettant la croissance des levures non-modifiées, correspondantes (levures dans lesquelles les mutations ont été introduites). La restauration de la croissance de la levure est mesurée par toute technique appropriée connue de l'Homme du métier, telle que notamment la spectrométrie (mesure de la densité optique de la culture, dans le cas de d'un milieu de culture liquide) ou la visualisation d'un halo de croissance à la surface du milieu de culture (milieu de culture gélosé).

Selon un mode de mise en oeuvre avantageux dudit procédé, ladite pathologie mitochondriale est un syndrome sélectionné dans le groupe constitué par : NARP, LHON (*Leber's Hereditary Ootic Neuropathy*)*,* MILS (*Maternally Inherited Leigh Syndrome*)*,* MERRF *(Myoclonic Epilepsy with Ragged-Red Fibers)* et HSP *(Hereditary Spastic Paraplegia*)*.*

Selon un autre mode de mise en oeuvre avantageux dudit procédé, la mise en contact de l'étape a) est réalisée par application d'un filtre contenant les molécules à tester, sur un milieu solide (milieu gélosé) ensemencé avec lesdites levures modifiées.

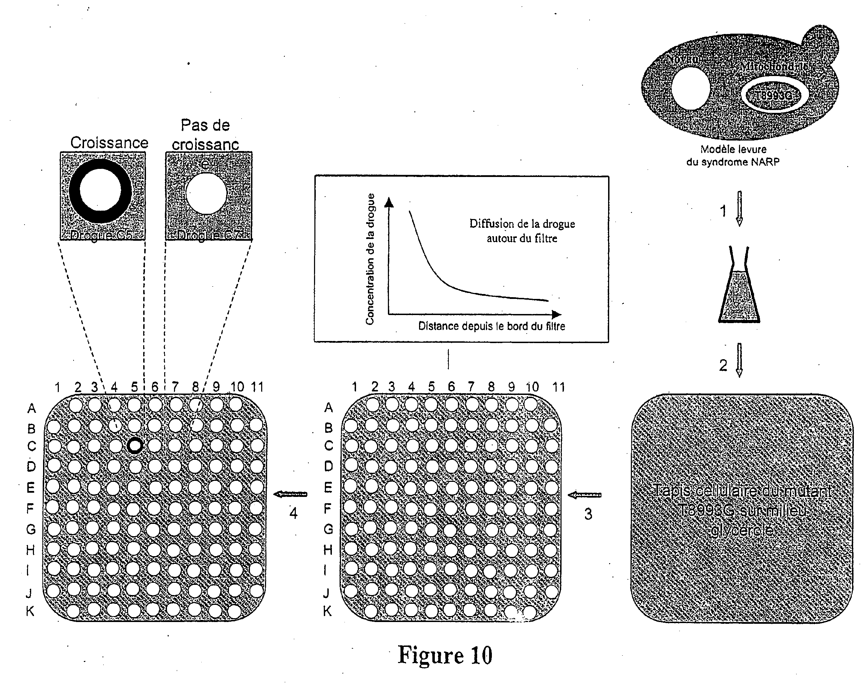

Par exemple, on peut envisager un test de criblage, selon le principe décrit dans Bach et al., précité. Pour cela, on peut étaler en tapis des cellules du mutant à la surface d'un milieu gélosé contenant une source de carbone non fermentescible (glycérol). On dispose ensuite de façon ordonnée sur le tapis cellulaire des filtres contenant chacun une quantité donnée d'une molécule. Les drogues diffusent dans le milieu de culture et un gradient de concentration s'établit autour des filtres. Si une molécule permet de contrecarrer les effets de la mutation il en résultera un halo de croissance autour du filtre (figure 10). Un tel criblage avec la levure est très simple à mettre en oeuvre et des dizaines de milliers de molécules peuvent ainsi être testées en quelques mois à peine.

Selon un autre mode de mise en oeuvre avantageux dudit procédé, ladite source de carbone non-fermentescible est sélectionnée dans le groupe constitué par le glycérol, l'éthanol et le lactate. Ces composés sont utilisés à des concentrations finales dans le milieu de culture, de l'ordre de 20g/l pour le glycérol et le lactate et 30 ml/l pour l'éthanol.

Parmi les types de molécules à tester on peut citer notamment :
- des petites molécules capables de se lier spécifiquement à l'ATP synthase, dans le voisinage de la région d'Atp6p modifiée par la mutation, et de restaurer le fonctionnement de l'ATP synthase. En effet, des changements discrets dans la protéine Atp6p permettent de compenser la présence de la mutation. En conséquence, de telles molécules pourraient restaurer le fonctionnement de l'ATP synthase en induisant un changement discret de conformation permettant de relâcher la contrainte causée par la mutation. Dans le cas de la mutation L183R, les résidus au voisinage de la mutation qui peuvent être ciblés par ces molécules comprennent les acides aminés en position 179, 180, 183 et 226. Ces molécules capables de restaurer le fonctionnement de l'ATP synthase sont potentiellement utilisables comme médicament pour le traitement du syndrome NARP.
- des suppresseurs métaboliques de la mutation, c'est-à-dire des molécules capables de corriger le déficit de synthèse d'ATP par la voie de la phosphorylation oxydative, causé par le dysfonctionnement du complexe ATP synthase mitochondrial, notamment des molécules capables de stimuler des voies alternatives de synthèse d'ATP dans les mitochondries. En effet, la surexpression, chez la souche mutante de levure t8993g, d'Odclp, une protéine de la membrane interne qui transporte de l'α-cétoglutarate dans la mitochondrie, conduit à une production accrue d'ATP dans les mitochondries par des phosphorylations d'ADP liées au substrat, au niveau du cycle de Krebs. Ainsi, les molécules qui conduisent à une dérégulation de l'expression d'Odclp pourraient augmenter la production intramitochondriale d'ATP au niveau du cycle de Krebs et ainsi compenser le dysfonctionnement de l'ATP synthase causé par la mutation t8993g. Ces molécules capables de restaurer la production d'ATP par les mitochondries sont potentiellement utilisables comme médicament pour le traitement, non seulement du syndrome NARP, mais aussi d'autres pathologies mitochondriales, liées notamment à un dysfonctionnement des complexes respiratoires, comme les syndromes LHON (*Leber's Hereditary Ootic Neuropathy*)*,* MILS (*Maternally Inherited Leigh Syndrome*)*,* MERRF *(Myoclonic Epilepsy with Ragged-Red Fibers)* et HSP *(Hereditary Spastic Paraplegia).*

Outre les dispositions qui précèdent, l'invention comprend encore d'autres dispositions qui ressortiront de la description qui va suivre, qui se réfère à des exemples illustrant la construction, la caractérisation génétique et moléculaire et l'utilisation de souches mutantes de levure portant l'équivalent de mutations du gène mitochondrial *ATP6* responsables du syndrome NARP chez l'homme, ainsi qu'aux dessins annexés dans lesquels :
- la figure 1 est une représentation schématique de l'appareil de transduction d'énergie mitochondrial et des gènes contrôlant sa formation.
- la figure 2 illustre la structure de l'ATP synthase et de la mutation t8993g associée au syndrome NARP. A. Structure de l'ATP synthase: la sous-unité Atp6p fait partie du secteur Fₒ de l'ATP synthase; l'ensemble Atp6p/Atp9p constitue le canal à protons. B. Mutation associée au syndrome NARP: la mutation t8993g associée au syndrome NARP entraîne le remplacement d'un résidu leucine conservé de la sous-unité Atp6p par l'arginine (en position 156 chez l'homme; 183 chez la levure). Dans les modèles de structure, ce résidu leucine est localisé dans la membrane interne à l'interface entre la sous-unité Atp6p et l'anneau de sous-unités Atp9p.
- la figure 3 illustre la construction et l'analyse génétique et moléculaire du mutant MR10 comprenant la délétion du gène mitochondrial *ATP6* et le remplacement de ce gène par le marqueur génétique *ARG8^{m}.*

A. Construction du mutant MR10. Le gène mitochondrial *ATP6* a été délété de la souche MR6 (*arg8::HIS3* [*rho⁺FY1679*] ; génome mitochondrial sauvage et gène nucléaire *ARG8* délété), et remplacé par le marqueur génétique *ARG8^{m},* une version mitochondriale du gène nucléaire *ARG8* qui code pour une protéine mitochondriale (Arg8p) intervenant dans la biosynthèse de l'arginine. La souche mutante ainsi obtenue est dénommée MR10 (*rho*⁺, Δ*atp6::ARG8m*).

B. Phénotype de croissance du mutant MR10, par rapport à la souche MR6. Contrairement à MR6, MR10 n'est plus capable de croître à partir d'une source de carbone non fermentescible (glycérol). En revanche, contrairement à MR6, MR10 est capable de croître en présence de glucose (sucre fermentescible), sans apport externe d'arginine.

C. Analyse en Southern-blot, de l'ADN génomique de la souche MR10 (*rho⁺,* Δ*atp6::ARG8m*)*,* par comparaison avec la souche MR6 (*rho*⁺) et une souche dépourvue d'ADN mitochondrial (*rho*°)*.* L'ADN digéré par *Swa* I a été hybridé avec des sondes radiomarquées spécifiques du gène *ATP6* ou *ARG8m.* Les signaux d'hybridation obtenus confirment le remplacement d'*ATP6* par *ARG8m* dans la souche MR10.

D. Complémentation génétique du mutant MR10. Un test de complémentation génétique du mutant MR10, par croisement avec une souche ρ⁻ synthétique (SDC30) ne contenant dans ses mitochondries que le gène *ATP6* confirme que le défaut de croissance respiratoire de MR10 est bien dû à l'inactivation du gène *ATP6.*

E. Analyse des protéines extraites des souches MR6 et MR10, en Western- blot avec des anticorps dirigés contre les protéines Atp9p et Atp6p. Une absence d'accumulation de la protéine Atp6p est observée dans la souche MR10.

F. Analyse des synthèses protéiques mitochondriales dans les souches MR6 (W.T.) et MR10 (Δatp6) par radiomarquage et électrophorèse en SDS-PAGE. On observe une absence de synthèse de la protéine Atp6p dans la souche MR10.

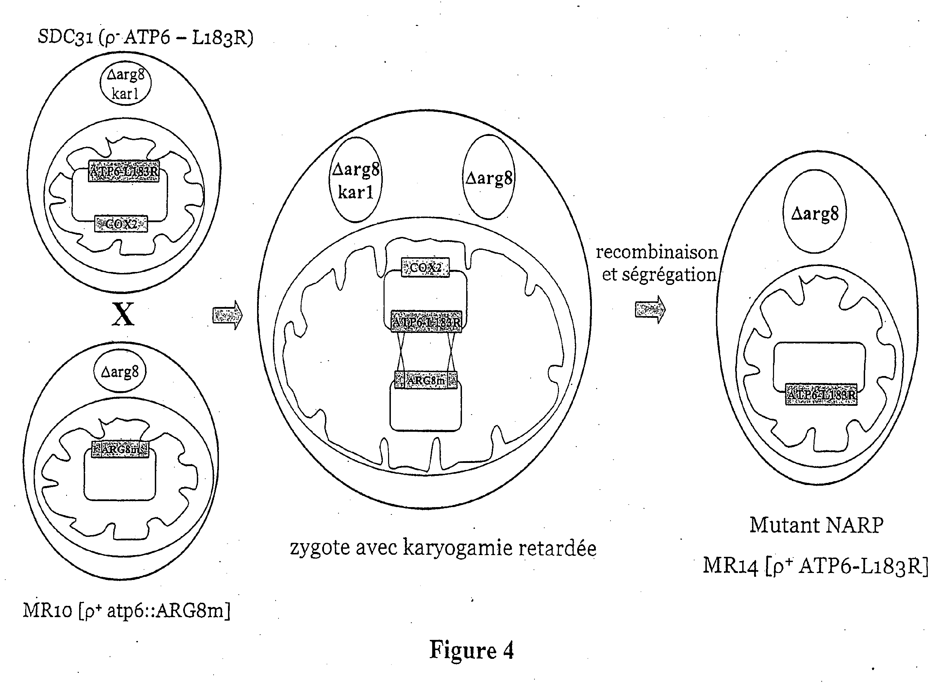

- la figure 4 illustre la stratégie utilisée pour introduire des mutations dans le gène *ATP6* de levure. L'équivalent de la mutation t8993g (tta₁₈₃ -> aga_{183;} L183>R) a été introduite dans le génome mitochondrial de la levure, par croisement de la souche SDC31 (ρ-, *ATP6-LI83R*) avec la souche MR10 (ρ+, *atp6::ARG8m*)*.* Dans les cellules zygotiques issues du croisement entre SDC31 et MR10, les mitochondries parentales fusionnent, et les ADN mitochondriaux de SDC31 et MR10 peuvent alors recombiner. Un double crossing-over conduit au remplacement d'*ARG8^{m}* par le gène *ATP6* portant la mutation tta -> aga. Grâce à la mutation *karl-1* dans le noyau de la souche SDC31, il a été possible d'obtenir des clones haploïdes ayant le noyau de la souche MR10 et le génome mitochondrial recombinant contenant le gène *ATP6* muté. Ayant perdu le gène *ARG8^{m},* ces derniers sont incapables de croître en absence d'arginine. Un de ces mutants NARP (*ρ+, ATP6-L183R*), dénommé MR14, a été retenu pour les analyses ultérieures.

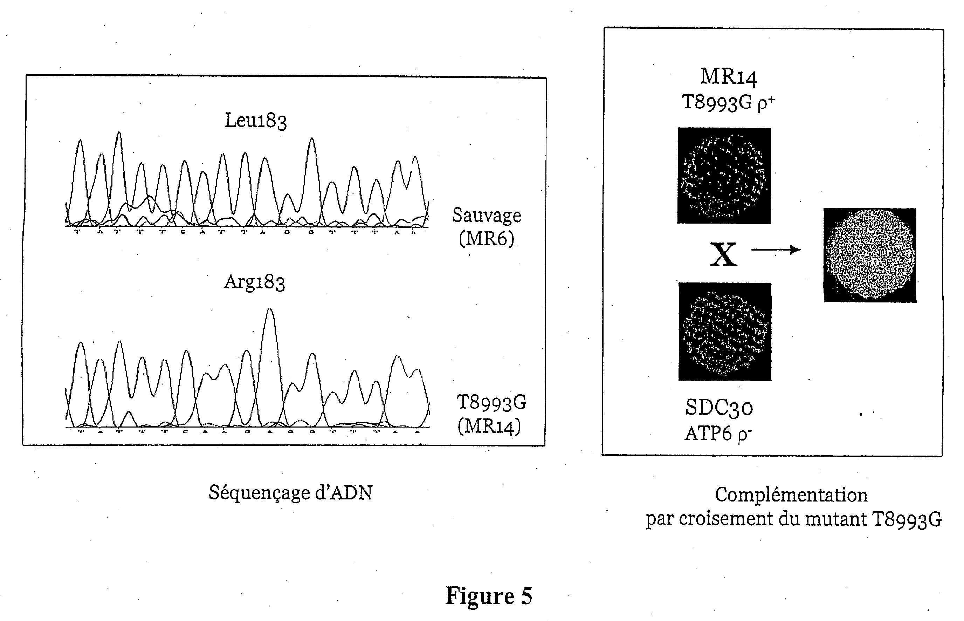

- la figure 5 illustre l'analyse moléculaire et génétique du mutant *t8993g* de levure (mutant MR14). A. Chromatographe de la séquence nucléotidique de la région du gène *ATP6* autour du codon 183. Chez le sauvage (MR6) ce codon (tta) spécifie un résidu leucine ; chez le mutant. (MR14), ce codon est modifié en aga qui spécifie l'arginine. B. Complémentation du mutant t8993g (MR14) par croisement avec une souche ρ⁻ synthétique (SDC30) ne contenant dans ses mitochondries que le gène *ATP6.* La croissance de MR14 sur glycérol est très ralentie. La souche SDC30 est incapable de croître sur ce milieu. La croissance sur glycérol est restaurée par le croisement, ce qui démontre que le phénotype de déficience respiratoire du mutant MR14 est bien dû à la mutation t8993g et à elle seule.

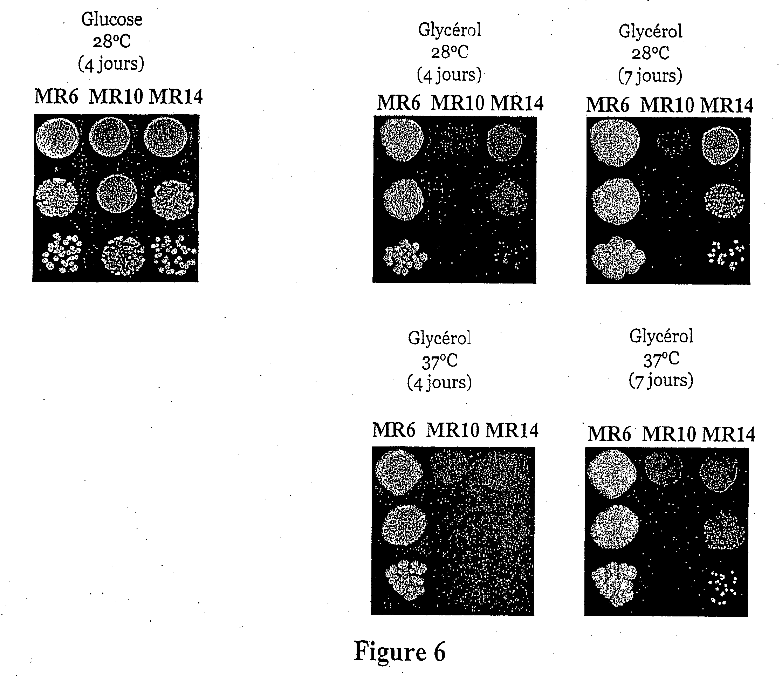

- la figure 6 montre que la mutation t8993g (L183>R) altère sévèrement la croissance respiratoire de la levure. La souche sauvage (MR6), le délétant Δ*atp6::ARG8^{m}* (MR10) et le mutant MR14 portant un équivalent tta₁₈₃>aga de la mutation t8993g, ont été cultivés à 28°C ou 37°C dans du milieu contenant une source de carbone fermentescible (glucose) ou non-fermentescible (glycérol). La souche mutante MR14 présente un fort défaut de croissance sur des milieux contenant une source de carbone non fermentescible (glycérol), tant à 28 °C qu'à 37 °C; on note ' seulement une très légère pousse sur glycérol après sept jours d'incubation alors que la croissance de la souche sauvage est déjà complète après à peine trois jours. En revanche, MR14 croît normalement par la voie fermentaire (glucose).

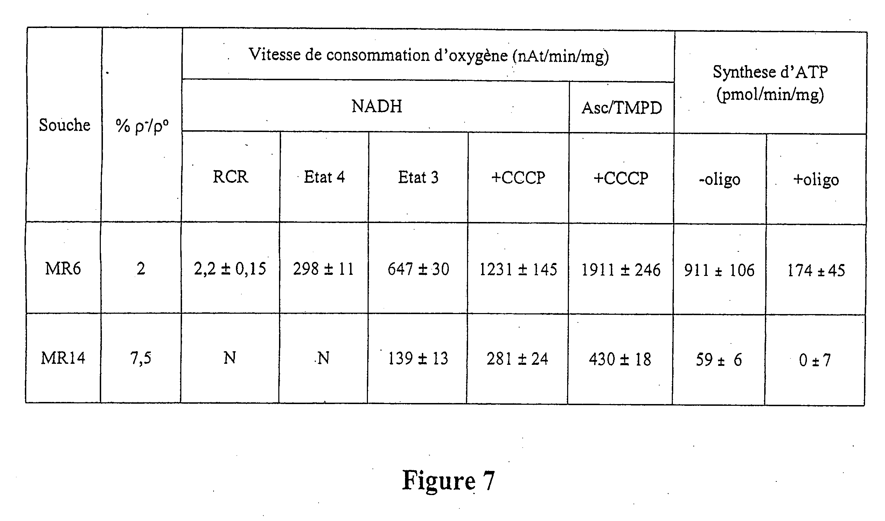

- la figure 7 illustre l'activité de la chaîne respiratoire et du complexe ATP synthase des souches sauvage (MR6) et mutante t8993g (MR14). Les mitochondries ont été isolées des souches sauvage (MR6) et mutante t8993g (MR14) cultivées en YPGALA. Les différents facteur ont été ajoutés aux concentration suivantes: protéines (0,15 mg/ml), NADH (état 4 ; 4 mM), ADP (état 3 ; 400 µM), oligomycine (oligo ; 6 µg/ml), CCCP (3 µM), Ascorbate (Asc ; 15 mM), TMPD (1,4 mM). Le coefficient respiratoire (RCR) est le rapport de la vitesse de respiration à l'état 3 sur la vitesse de respiration à l'état 4. N : non applicable. Les pourcentages de petites ρ-/ρ° dans les cultures sont indiqués. Dans le cas du mutant MR14, la vitesse de la respiration à l'état 4 est près de trois fois plus faible comparativement à la vitesse de respiration à l'état 4 mesurée pour MR6 (81 contre 298 O.min⁻¹.mg⁻¹).

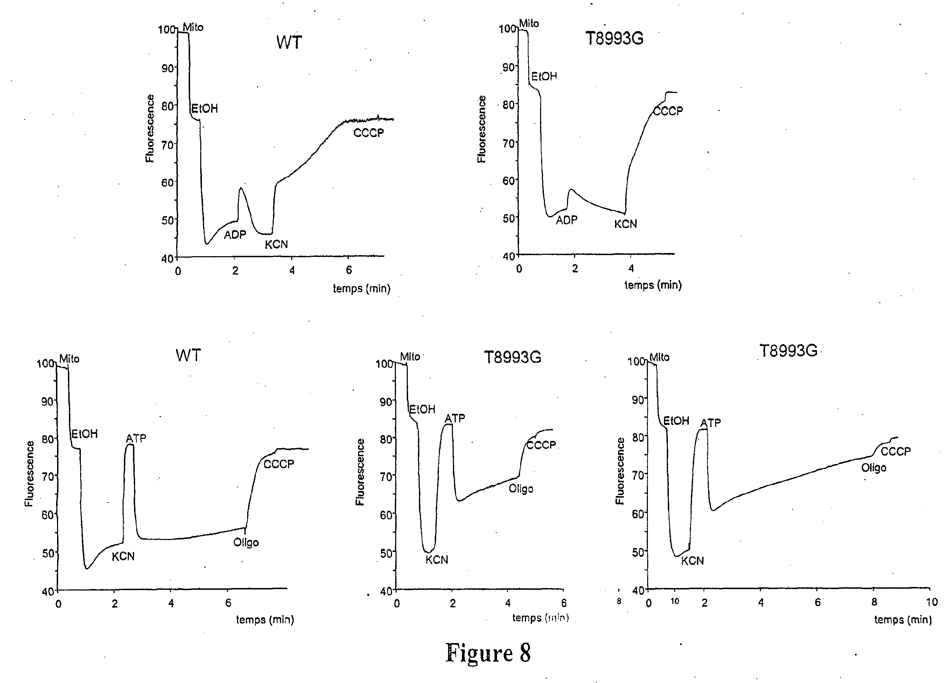

- la figure 8 illustre l'analyse de l'énergisation de la membrane mitochondriale interne par fluorométrie avec de la rhodamine 123. Différents facteurs ont été ajoutés aux mitochondries intactes de la souche sauvage MR6 (WT) et du mutant MR14 (T8993G): rhodamine 123 (0,5 µg/ml), protéines mitochondriales (Mito ; 0,3 mg/ml), éthanol (EtOH ; 10 µl), oligomycine (oligo ; 6 µg/ml), cyanure de potassium (KCN ; 0,2 mM), et carbonyl cyanide m-chlorophenylhydrazone (CCCP ; 3 mM).

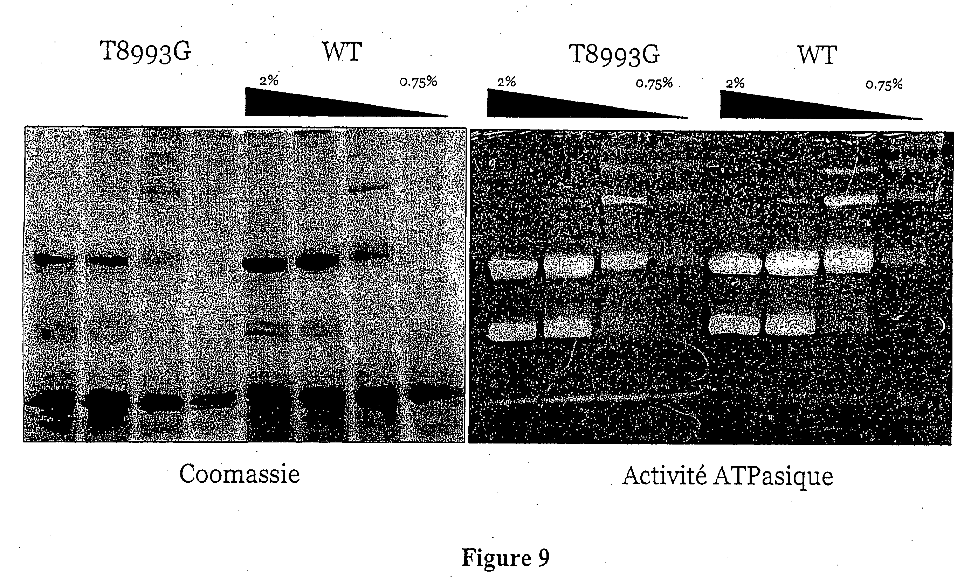

- la figure 9 illustre l'analyse du complexe ATP synthase par la technique BN-PAGE. Des mitochondries de la souche sauvage (MR6) et du mutant t8993g ont été isolées puis solubilisées avec de la digitonine aux concentrations indiquées. Après centrifugation, les complexes ont été séparés par BN-PAGE et les gels ont été colorés au bleu de Coomassie ou incubés avec de l'ATP-Mg²⁺ et du Pb²⁺ pour révéler l'activité ATPasique.
- la figure 10 illustre le criblage de molécules actives contre le syndrome NARP, à l'aide des mutants de levure. Etape 1: culture du mutant en milieu glucose. Etape 2 : les cellules mutantes sont étalées en tapis à la surface d'un milieu gélosé contenant du glycérol comme source de carbone. Etape 3: des filtres contenant chacun une quantité définie de l'une des molécules à tester sont disposés sur la boîte de Pétri, les molécules diffusent dans le milieu et établissent un gradient de concentration autour des filtres. Etape 4 : les boîtes sont incubées ; on voit apparaître un halo de croissance autour des filtres contenant une substance capable de contrecarrer les effets de la mutation (drogue C5).

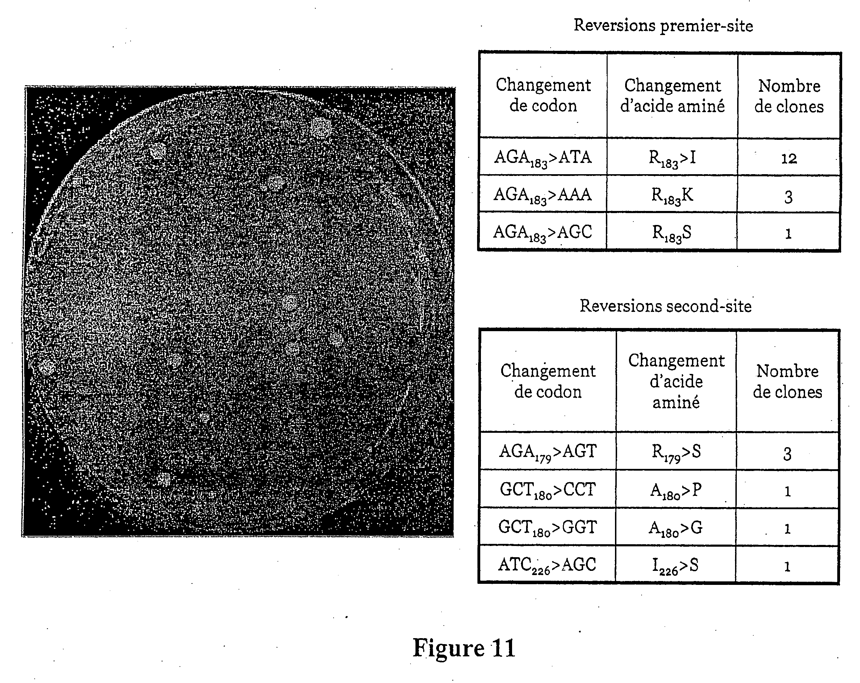

- la figure 11 illustre la séquence des révertants du mutant t8993g. Des cellules du mutant t8993g de levure ont été étalées en tapis dense à la surface d'un milieu nutritif contenant une source de carbone non fermentescible (glycérol, milieu N3), c'est-à-dire des conditions qui ne permettent pas la croissance du mutant t8993g. On voit apparaître après quelques jours d'incubation des clones révertants ayant récupéré une capacité respiratoire suffisante. Les gènes *ATP6* de ces révertants ont été amplifiés par PCR et séquences. Les suppresseurs, dits 'premier-site' conduisent au remplacement de l'arginine 183 par un autre acide aminé que la leucine présente dans la séquence sauvage d'Atp6p, à savoir la lysine, l'isoleucine, ou la sérine. Les suppresseurs dits 'second-site' conduisent au remplacement du résidu arginine 179 par la sérine, du résidu alanine 180 par la proline ou la glycine, ou du résidu isoleucine 226 par la sérine.

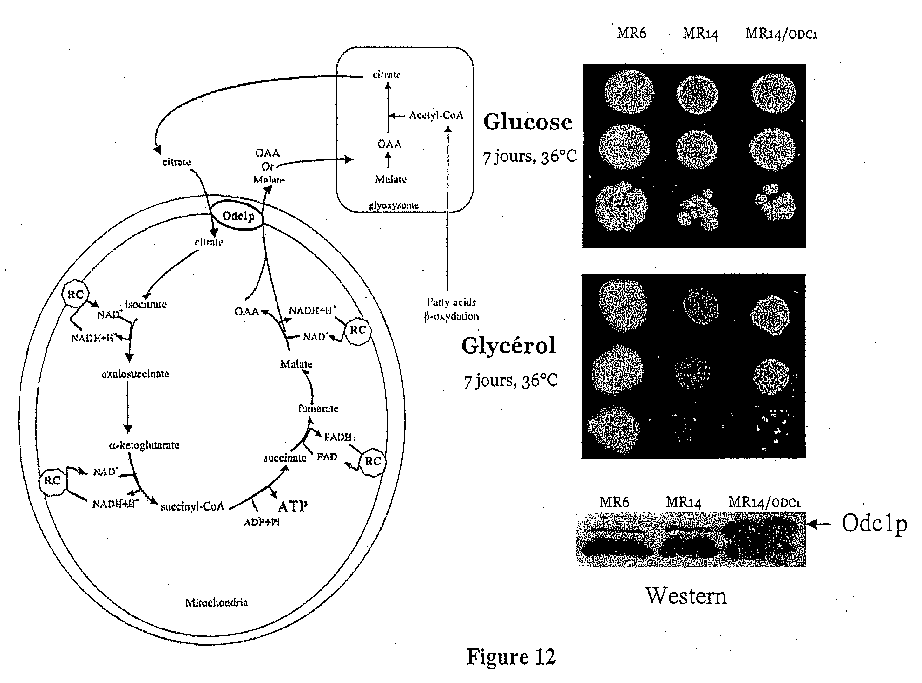

- la figure 12 illustre la suppression métabolique du mutant t8993g par la surexpression d'Odc1p.

A. Représentation schématique du mécanisme de suppression métabolique. Dans les cellules entières, un contre échange citrate/malate ou oxaloacétate à travers la membrane mitochondriale interne est catalysé par Odc1p. Le citrate produit au cours du cycle glyoxylate est transporté dans les mitochondries par Odclp (Palmieri et al., J. Biol., Chem., 2001, 276, 1916-1922) et peut entrer dans le cycle TCA (cycle de Krebs) et conduire à la production de succinate, couplé à une phosphorylation d'ADP au niveau du substrat. Le malate ou l'oxaloacétate peuvent ensuite être transportés vers le cytosol via Odclp et entrer dans le cycle glyoxylate. Ce cycle de réactions peut se perpétuer du fait de la production d'acétyle-CoA via la dégradation d'acides gras qui est accrue du fait de la prolifération des péroxysomes dans les cellules où la réponse rétrograde est activée (pour revue, voir Butow, R.A. and Avadhani, N.G. Moll.Cell 2004, 14, 1-15). RC désigne la chaîne respiratoire.

B. Complémentation partielle de la mutation t8993g par la surexpression d'Odcpl. Les souches sauvage (MR6), mutante t8993g (MR14), et mutante t8993g surexprimant Odclp (MR14/ODC1) ont été cultivées pendant une nuit dans un milieu glucosé (YPGA). Les cultures ont été diluées de façon sériée, et une goutte de chaque dilution a été déposée sur du milieu YPGA et du milieu contenant du glycérol comme source de carbone (milieu N3). Les boîtes ont en suite été incubées à 36 °C et photographiées après 7 jours d'incubation. L'analyse des protéines extraites des-souches MR6, MR14 et MR14/ODC1, par Western blot avec des anticorps dirigés contre Odclp, confirme qu'Odcp1 est surexprimée dans la souche MR14/ODC1.

### Exemple 1: Introduction dans le génome mitochondrial de S. cerevisiae des mutations du gène ATP6 responsables du syndrome NARP chez l'homme

Les acides nucléiques sont manipulés selon les méthodes classiques, à l'aide des protocoles standards tels que décrits dans: Current Protocols in Molecular Biology (Frederick M. AUSUBEL, 2000, Wiley and son Inc, Library of Congress, USA*)* et Molecular Cloning: A Laboratory Manual, Third Edition, (Sambrook et al, 2001, Cold Spring Harbor, New York: Cold Spring Harbor Laboratory Press*).*

### 1) Matériels et méthodes

### 1.1) Techniques de biologie moléculaire et de génétique

### a) Souches de levure, de bactérie, plasmides

Les souches de levure, de bactérie et les plasmides suivants ont été utilisés:
- MR6: *mat* a, *ade2, leu2, ura3, trp1, his3, arg8::HIS3* [*rho⁺FY1679*]
- DFS160: *mat* α*, ade2, leu2, ura3, kar1-1, Δarg8:: URA3* [*rho⁰*]
- SDC30: *mat α, ade2, leu2, ura3,* Δ*arg8:: URA3* [*rho*⁻ *ATP6, COX2*]
- NB40-3c : *mat α, lys2, leu2, ura3 his3deltaHinDIII, argB::IzisG [rho+ cox2-62]*
- *E. coli* XL1-Blue: *rec.41, endaA1, gyrA9, thi-1, hsdR17, supE44, relA1, lac* [*F' proAB lac I^{q}Z*Δ*M15 Tn 10 (Tet^{r})*]
- pJM2 (Mulero J.J. & Fox T.D., Mol. Biol. Cell., 4, 1327-1335).

### b) Milieux de culture pour levure

Les souches de levure sont cultivées dans les milieux suivants :
- YPGA: Yeast extract 1 % (p/v), Bactopeptone 1 %, glucose 2 %, adénine 20 mg/l
- YPGALA: Yeast extract 1 %, Bactopeptone 1 %, galactose 2 %, adénine 30 mg/l
- N3: Yeast extract 1 %, Bactopeptone 1 %, Na₂HPO₄ 10 mM, KH₂PO₄ 40 mM, glycérol 20 ml/l; 2 % d'agar sont incorporés pour la solidification des milieux.

Les bactéries sont cultivées en milieu LB: NaCl 5 g/l, Yeast extract 0,5 %, NaOH 0,3 N, Bactotryptone 1 %.

### c) Amplification d'ADN par réaction de polymérisation en chaîne (PCR)

Les amplifications d'ADN par PCR ont été réalisées avec la polymerase pFU (STRATAGENE), dans 50 µl d'un tampon fourni par le fabriquant contenant 100 ng de l'ADN portant la séquence nucléotidique à amplifier, 25 picomoles de chacune des deux amorces d'amplification, 200 µM de chaque dNTP, et une unité d'enzyme. Le programme suivant a été utilisé: [95 °C-2 min; 55 °C-30 sec; 72 °C-1 min 30 sec] x 1; [95 °C-30 sec; 55 °C-45 sec; 72 °C-1 min 30 sec] x 28; [95 °C-30 sec; 55 °C-30 sec; 72 °C-15 min] x 1.

### d) Purification de fragments d'ADN

Les fragments d'ADN ont été séparés en gel agarose 1 %-TBE (89 mM acide borique, 25 mM EDTA, 89 mM Tris-base) contenant 0,5 µg/ml de bromure d'éthidium pour leur visualisation sous UV (254 nm). Les fragments d'intérêt ont été purifiés à l'aide du Kit QIAquick Gel Extraction® (QIAGEN), en suivant les instructions du fournisseur.

### e) Déphosphorylation des extrémités de fragments d'ADN

Les extrémités de fragments d'ADN ont été déphosphorylées dans un tampon 20 mM Tris-HCl pH 8, contenant 2 unités de phosphatase alcaline (Calf Intestinal Phosphatase, BIOLABS), à 37°C pendant une heure. La réaction est arrêtée par l'ajout de 2 µl de 0,5 M EDTA pH 8, et 15 µl de SDS 10 %. Le mélange est incubé 15 min à 70 °C puis traité au phénol, puis au chloroforme. L'ADN de la phase aqueuse est précipité par l'ajout de 2 volumes d'éthanol, 1/10 de volume d'acétate de sodium 3 M, pH 5.2. Le précipité est lavé à l'éthanol 70 %, puis séché sous vide et repris dans de l'eau.

### f) Ligation de fragments d'ADN

La ligase du phage T4 a été utilisée pour catalyser la formation de liaisons phosphodiesters entre fragments d'ADN, selon les recommandations du fournisseur (GIBCO).

### g) Mutagénèse

La mutagénèse dirigée est réalisée à partir du gène cloné dans un plasmide, à l'aide du kit Gene Editor In Vitro Site-Directed Mutagenesis System® (PROMEGA), en suivant les instructions du fournisseur.

### h) Transformation des bactéries par électroporation

La souche d'*E*. *coli* XL1-Blue est cultivée en milieu LB, à 37°C, sous agitation, jusqu'à une D.0.₆₀₀ₙₘ de 0,5 à 1. Les cellules sont lavées plusieurs fois avec de l'eau stérile à 4°C, puis concentrées dans une solution de glycérol à 10 % (p/v). Les cellules ainsi préparées sont aliquotées et conservées à -80°C_ L'électroporation est réalisée à l'aide de l'appareil ECM 395 (BTX). 40 µl de cellules compétentes sont mélangées à 100 ng d'ADN plasmidique, soumises à une décharge électrique (2,5 kV), puis du milieu LB est ajouté. La suspension de cellules est ensuite incubée 1 heure à 37 °C puis étalée sur du milieu LB contenant 100 µg/ml d'ampicilline, pour la sélection des transformants.

### i) Réparation d'ADN plasmidique

Les plasmides sont amplifiés dans la souche d'*E. coli* XL1-Blue transformée par électroporation, comme précisé en h). L'ADN plasmidique est ensuite extrait des bactéries, à l'aide du Plasmid Midi Kit® (QIAGEN), en suivant les recommandations du fournisseur.

### j) Transformation classique des cellules de levure (transformation nucléaire)

La veille de l'expérience, des gouttes d'une culture fraîche de cellules à transformer sont déposées sur du milieu YPGA. Le lendemain, des cellules sont prélevées avec une pointe de cure dent et mélangées à 100 µl d'un tampon 0,2 N acétate de lithium, pH 5, 40 % polyéthylène glycol 4000, 100 mM DTT, contenant 50 µg d'ADN carrier préalablement dénaturé à 100 °C pendant 20 min, et 50 ng à 1 µg d'ADN du plasmide à introduire dans les cellules. Après 30 min d'incubation à 45 °C, les cellules sont lavées avec du tampon Ringer, puis étalées sur un milieu approprié à la sélection des transformantes.

### k) Obtention de transformants mitochondriaux

L'ADN plasmidique est introduit dans les mitochondries d'une souche de levure (DFS160) entièrement dépourvue d'ADN mitochondrial (ρ°), par bombardement biolistique, à l'aide du système 'Biolistic PDS-1000/HeTM (BIO-RAD), selon le protocole précédemment décrit (Bonnefoy, N. et Fox, T.D., Methods Cell Biology, 2001, 65, 381-396).

### l) Croisement des souches de levure et isolement des recombinants cytoductants) haploïdes

La procédure utilisée est telle que décrite dans (Bonnefoy, N. et Fox, T.D., Methods Cell Biology, 2001, 65, 381-396).

### m) Analyse d'ADN génomique de levure par Southern Blot.

Les extractions d'ADN génomique de levure ont été réalisées à partir de cellules cultivées une nuit dans du milieu YPGA. Les cellules sont récoltées par centrifugation puis reprises dans 0,2 ml de tampon 0,1 M NaCl contenant 2 % de Triton-X100, 1 % de SDS et 0,2 ml d'un mélange phénol/chloroforme/alcool isoamylique (50/48/2). 500 µl de billes de verre (diamètre 0,45 mm) sont ajoutés. Le mélange est vortexé pendant 2 min, puis 0,2 ml de tampon 10 mM TrisHCl pH 8, 1 mM EDTA sont ajoutés. La phase aqueuse est ensuite séparée par centrifugation (5 min à 10000 x g) et les acides nucléiques qu'elle contient sont précipités par l'ajout de 20 µl d'acétate d'ammonium 8 M et 1 ml d'éthanol. Le précipité est récolté par centrifugation, lavé avec de l'éthanol 70 %, puis repris et incubé 15 min à 37 °C, dans 400 µl de 10 mM TrisHCl pH 8, 1 mM EDTA, contenant 30 µg de Rnase A. L'ADN de l'échantillon est précipité par l'ajout de 2,5 volumes d'éthanol et 1110 de volume d'acétate de sodium 3 M à pH 5,2. Il est lavé avec de l'éthanol 70 %, séché, et finalement repris dans 50 µl d'eau.

L'ADN est digéré avec les enzymes de restriction appropriées et les fragments d'ADN résultants sont séparés par électrophorèse en gel d'agarose, transférés sur une membrane de nitrocellulose et hybridés avec une sonde spécifique radiomarquée. La membrane est ensuite auto-radiographiée pour la visualisation des fragments d'intérêt.

### n) Test de complémentation génétique

Le mutant de levure déficient pour la respiration et la souche ρ⁻ synthétique ne contenant dans ses mitochondries que le gène *ATP6* (souche SDC30) sont cultivées séparément, pendant une nuit dans un milieu glucosé (YPGA). Un croisement 'goutte sur goutte' est ensuite réalisé sur milieu YPGA. Après une nuit d'incubation; le croisement est répliqué au velours sur un milieu glycérolé (N3). La présence de cellules issues du croisement, capables de croître sur glycérol, signe la présence de recombinants qui ont reconstitué un génome mitochondrial sauvage par recombinaison des ADN mitochondriaux des deux souches.

### 1.2) Techniques de biochimie

### a) Extraction des protéines totales de levures

Les cellules sont cultivées en milieu YPGALA et récoltées lorsque l'absorbance est de 2 unités de densité optique (D.O.) à 650 nm. Les cellules sont lavées deux fois avec de l'eau puis reprises avec de l'eau à une densité de 10 D.O./ml. La lyse des cellules est obtenue par addition de 150 µl de tampon 1,85 M NaOH, 7,4 % β-mercaptoéthanol à 1 ml de suspension cellulaire. Une incubation de 10 min à 4 °C est réalisée puis 150 µl d'acide trichloroacétique (TCA) 3M sont ajoutés pour la précipitation des protéines. Une nouvelle incubation de 10 min à 4 °C est réalisée puis le mélange est centrifugé 5 min à 10000 x g. Le culot est lavé avec de l'acétone à - 20 °C pour éliminer toute trace de TCA. Ensuite, le culot est repris dans 250 µl de SDS 5%, traité par les ultrasons, puis incubé 5 min à 100 °C. L'échantillon protéique est finalement récolté par centrifugation (5 min à 10000 x g).

### b) Analyse des synthèses protéiques mitochondriales par incorporation de méthionine et cystéine radiomarquées avec du ³⁵S

La procédure est celle décrite par Lefebvre-Legendre et al. J; Biol. Chem., 2001, 276, 6789-6796. L'expérience est réalisée avec des cellules en phase exponentielle de croissance dans du milieu YPGALA. Les cellules sont lavées avec de l'eau puis reprises et incubées pendant 40 min dans du LSM 1 X contenant 1 % de galactose et les nutriments correspondant aux marqueurs d'auxotrophie de la souche à analyser (LSM 10X : H₃Bo₃ 5. mg/l, CuCl₂ 0,33 mg/l, KI 1 mg/l, MnCl₂ 4H₂O 5,2 mg/l, Na₂MoO₄ 2H₂O 2,35 mg/l, ZnCl₂ 3,4 mg/l, FeCl₃ 6H₂O 2 mg/l, pantothénate de calcium 20 mg/l, chlorure de thiamine 20 mg/l, pyridoxine 20 mg/l, acide nicotinique 5 mg/l, biotine 0,2 mg/l, mesoinositol 200 mg/l, (NH₄)H₂PO₄ 54 g/l, MgCl₂ 6H₂O 3,65 g/l, NH₄Cl 14,58 g/l, KH₂PO₄ 9 g/l, NaCl 0,9 g/l, CaCl₂ 2H₂O 1,188 g/l. Cette étape vise à carencer les cellules en cystéine et en méthionine. De la cycloheximide est ensuite ajoutée à une concentration finale de 250 µg/ml, pour inhiber les synthèses protéiques cytosolique extramitochondriale; la radioactivité n'est donc incorporée que dans les protéines codées par le génome mitochondrial. Après 5 min, on ajoute 0,5 mCi de promix (L-[³⁵S] méthionine et L-[³⁵S] cystéine, AMERSHAM) et l'échantillon est incubé pendant 10 min à 30°C. Une solution de casamino acids 1 % est ajoutée pour arrêter l'incorporation des acides aminés radiomarqués et permettre la terminaison des produits de traduction. Les cellules sont récoltées et lavées 2 fois successivement dans la solution de casamino acids 1 % puis reprises dans du tampon 0,25 M mannitol, 20 mM Tris-sulfate pH 7,4, 1 mM EDTA, 1 mM PMSF. Des billes de verre (0,45 mm de diamètre) sont ajoutées et les échantillons sont vortexés pendant 5 min pour broyer les cellules. Le broyat est centrifugé à basse vitesse (5 min à 750 x g à 4°C) pour l'élimination des débris cellulaires. Le surnageant est centrifugé a haute vitesse (12000 x g à 4°C pendant 20 min) pour la récolte des membranes mitochondriales. La radioactivité des échantillons est mesurée et les protéines radiomarquées qu'ils contiennent sont ensuite analysées en gel SDS-PAGE suivi d'une autoradiographie du gel.

### c) Electrophorèse, immuno-détection et dosage des protéines.

L'électrophorèse de protéines en gel d'acrylamide dénaturant (SDS-PAGE) a été réalisée selon la procédure de Laemmli, Nature, 1970, 227, 680-685. La technique d'électrophorèse en gel non-dénaturant (BN-PAGE) utilisée est celle décrite par Schagger et al., Anal. Biochem., 1994, 217, 220-230. L'activité ATPasique des gels BN-PAGE a été détectée par la méthode décrite par Grandier-Vazeille et Guerin, Anal. Biochem., 1996, 242, 248-254. La détection des protéines sur membrane de nitrocellulose avec des anticorps spécifiques a été réalisée avec la technique décrite par Paumard et al., EMBO J., 2002, 21, 221-230. Les complexes antigène-anticorps ont été révélés avec le kit ECL+® (AMERSHAM). Les protéines ont été dosées avec la méthode de Lowry et al., J. Biol. Chem., 1951, 193, 265-275:

### 2) Résultats

### a) Construction de la levure mutante MR10 par délétion du gène mitochondrial ATP6 et remplacement par le marqueur génétique ARG8^{m}

Le gène *ATP6* est chez la levure, comme chez l'homme, localisé dans le génome mitochondrial. Chez la levure, *ATP6* fait partie d'une unité de transcription polycistronique contenant les gènes *COXI* (codant pour une sous-unité du complexe IV), *ATP8* (codant pour une sous-unité de l'ATP synthase), et, dans certaines souches, *ENS2* qui code pour une endodésoxyribonucléase (Figure 3A). Chez l'homme, la principale mutation responsable du syndrome de NARP est un simple changement de nucléotides (t vers g) en position 8993 du génome mitochondrial (désigné par t8993g). Ce changement conduit au remplacement par l'arginine du résidu leucine 156 de la protéine Atp6p humaine (Figure 2). Ce résidu leucine est conservé dans la protéine Atp6p de la levure mais dans une position différente (en 183). Pour obtenir le remplacement de ce résidu par l'arginine chez la levure, un double changement de nucléotides est nécessaire : tta -> aga. En outre, d'autres mutations associées au syndrome NARP ont été détectées chez l'homme: t8993c, t9176g, t9176c et t8851c (Tableau I).

Pour faciliter l'introduction de ces mutations dans *ATP6,* une souche de levure (MR10) a tout d'abord été construite à partir de la souche MR6 (génome mitochondrial sauvage et gène nucléaire *ARG8* délété ; *arg8::HIS3 [rho*⁺*FY1679]).* Dans MR10, le gène *ATP6* a été délété et remplacé par le gène *ARG8m,* un marqueur génétique indépendant de la fonction respiratoire (Bonnefoy, N. et Fox, T.D., Methods Cell Biology, 2001, 65, 381-396). Le gène *ARG8m* est une version mitochondriale (version recodée) du gène nucléaire *ARG8* qui code pour une protéine mitochondriale (Arg8p) intervenant dans la biosynthèse de l'arginine.

Une cassette d'inactivation du gène *ATP6* par *ARG8*^{m}*. (atp6 ::ARG8m)* a été construite par PCR, avec les oligonucléotides :
- ATP6-PRO (SEQ ID NO : 3) :
   gcgggatcctttattatagtttaatactccatatgtaaattattttattttataattttattttataatttaagcatatacagcttcg, et
- ATP6-Ter (SEQ ID NO : 4) :
   gcctagataataagatataattatgattaattattataagttatatagttttataaatttataattattatgacacatttagaaagaa.

Ces oligonucléotides portent à leur extrémité 5', respectivement, un site *BamH* I et *Xba* I. Le produit PCR a été digéré par *BamH* I et *Xba* I puis cloné aux sites *BamH* I et *Xba* I du plasmide pJM2, précédemment décrit (Mulero, J.J. & Fox, T.D., Mol. Cell. Biol., 1993, 4, 1327-1335). Le plasmide résultant a été introduit par bombardement dans les mitochondries *rho°* de la souche DFS160. La souche résultante ρ⁻ synthétique a été croisée avec la souche MR6. Dans les cellules zygotiques, les mitochondries parentales fusionnent et les ADN mitochondriaux parentaux peuvent alors recombiner. Un double crossing-over conduit au remplacement d'*ATP6* par *ARG8^{m}.* La souche DFS160 porte la mutation nucléaire *kar1-1* qui a pour effet de retarder la fusion des noyaux et conduit à l'obtention de clones haploides (Bonnefoy, N. et Fox, T.D., 2001, Methods Cell Biology, 2001, 65, 381-396). Des clones haploides ayant le noyau de la souche MR6 et le génome mitochondrial recombinant *Δatp6 ::ARG8m* ont ainsi été obtenus. L'un de ces clones, dénommé MR10 (*mat a, ade2, leu2, ura3, trp1, his3, arg8::HIS3* [*rho+ FY1679; Δatp6::ARG8m* ]), a été retenu pour les analyses ultérieures.

### b) Analyse génétique et moléculaire du mutant MR10

L'analyse du phénotype de croissance du mutant MR10, par rapport à la souche MR6 (figure 3B), montre que MR10 n'est plus capable de croître à partir d'une source de carbone non fermentescible (glycérol). En revanche, contrairement à MR6, MR10 est capable de croître en présence de glucose (sucre fermentescible), sans apport externe d'arginine.

L'analyse de l'ADN génomique des souches MR6, MR10 et d'une souche dépourvue d'ADN mitochondrial *(rho°)* digéré par *Swa* I, par Southern-blot avec des sondes radioamarquées spécifiques du gène *ATP6* ou *ARG8m,* confirme le remplacement *d'ATP6* par *ARG8m* dans la souche MR10 (figure 3C).

La complémentation génétique du mutant MR10, par croisement avec une souche ρ⁻ synthétique (SDC30) ne contenant dans ses mitochondries que le gène *ATP6,* confirme que le défaut de croissance respiratoire de MR10 est bien dû à l'inactivation du gène *ATP6* (figure 3D).

L'analyse des protéines extraites des souches MR6 et MR10, par Western- blot avec des anticorps dirigés contre les protéines Atp9p et Atp6p, démontre l'absence d'accumulation de la protéine Atp6p dans la souche MR10 (figure 3E).

L'analyse des synthèses protéiques mitochondriales dans les souches MR6 et MR10 par radiomarquage et électrophorèse en SDS-PAGE, démontre l'absence de synthèse de la protéine Atp6p dans la souche MR10 (figure 3F).

### c) Construction de mutants du gène ATP6 de levure portant des mutations responsables du syndrome NARP chez l'homme.

L'équivalent de chacune des cinq mutations responsables du syndrome NARP chez l'homme (Tableau I) a été introduit séparément dans le gène *ATP6* de levure cloné dans le plasmide pJM2. Le gène *ATP6* de levure a été amplifié avec les oligonucléotides ATP6-up (SEQ ID NO: 5: gcggaccccaaaggaggag) et ATP6-down (SEQ ID NO: 6: cgggatcccagtggggaaggagtgaggt) qui portent chacun un site de restriction *BamH* I à leur extrémité 5'. Le produit PCR a été digéré par *BamH* I puis cloné au site *BamH* 1 de pJM2, pour donner le plasmide pSDC21. Les mutations ont ensuite été introduites séparément dans le gène *ATP6* cloné dans le plasmide pSDC21. Les cinq plasmides portant les différentes mutations (t8993g (pSDC22), t8993c, t9176g, t9176c et t8851c) ont ensuite été introduits séparément par bombardement biolistique, dans les mitochondries d'une souche de levure (DFS160) entièrement dépourvue d'ADN mitochondrial (ρ°). Les cinq souches rho⁻ synthétiques résultantes contenant chacune l'un des différents plasmides mutés dans leurs mitochondries ont été isolées; la souche contenant le plasmide pSDC22 dans ses mitochondries a été appelée SDC31 (*mat α, ade2, leu2, ura3, Δarg8::URA3 [rho⁻ atp6 t8993g, COX2*] ou *(ρ⁻, ATP6-L183R)).*

### d) Construction des mutants de levure par introduction du gène ATP6 muté dans le génome mitochondrial du mutant MR10

Les cinq mutations ont ensuite été introduites dans le génome mitochondrial de la levure, par croisement des cinq transformants mitochondriaux (ρ-*, ATP6-L183R* (SDC31); ρ*-, ATP6-L183P;* ρ-*, ATP6-L247R;* ρ*-, ATP6-L247P;* ρ*-, ATP6-L136R)* avec la souche MR10 (ρ+, *Δatp6::ARG8m*)*.* Dans les cellules zygotiques issues du croisement entre les souches rho⁻ synthétiques (SDC31 dans le cas de la mutation L183R) et MR10, les mitochondries parentales fusionnent (Figure 4). Cela aboutit à la mise en contact des ADN mitochondriaux des souches rho⁻ synthétiques (SDC31) et MR10 qui peuvent alors recombiner. Un double crossing-over conduit au remplacement *d'ARG8"'* par le gène *ATP6* portant l'une des cinq mutations. Grâce à la mutation *kar1-1* dans le noyau des souches rho⁻ synthétiques (SDC31) il a été possible d'obtenir des clones haploïdes ayant le noyau de la souche MR10 et le génome mitochondrial recombinant contenant le gène *ATP6* muté. Ayant perdu le gène *ARG8^{m},* ces derniers sont incapables de croître en l'absence d'arginine. Pour chacune des mutations, l'un des recombinants (MR14, RKY20-1, RKY-25-4, RKY38-1 et RKY39-1; Tableau I) a été retenu pour les analyses ultérieures.

### e) Analyse génétique et moléculaire des mutants du gène ATP6

La région du gène *ATP6* autour de la mutation a été séquencée chez MR6 (sauvage) et chez les mutants. Le chromatographe (figure 5A) montre la présence chez le sauvage, d'un codon (tta) qui spécifie un résidu leucine ; chez le mutant (MR14), ce codon est modifié en aga qui spécifie l'arginine.

La croissance des mutants t8893g (MR14), t9176g (RKY25-4) et t8851c (RKY39-1) sur glycérol est très ralentie (Figure 5B, Tableau I). La souche SDC31 (ρ⁻, *ATP6-L183R*) est incapable de croître sur ce milieu (Figure 5B). En revanche, les mutations t8993c (RKY20-1), et t9176c (RKY38-1) n'ont pas d'incidence marquée sur la croissance respiratoire de la levure (Tableau I).

Le test de complémentation de la mutation par croisement avec une souche ρ⁻ synthétique (SDC30) ne contenant dans ses mitochondries que le gène *ATP6,* montre que la croissance sur glycérol est restaurée par le croisement (figure 5B). Ce test démontre que le phénotype de déficience respiratoire du mutant est bien dû à la mutation et à elle seule.

### Exemple 2: Analyse des effets des mutations du gène ATP6 chez la levure

### 1) Matériels et méthodes

### a) Extraction des mitochondries de levure

La méthode utilisée pour l'extraction des mitochondries est celle décrite par Guérin et al, Methods Enzymol., 1979, 55, 149-159. Les levures, en phase exponentielle de croissance dans du milieu YPGALA, sont récoltées par centrifugation (5 min à 2000 x g) lavées avec de l'eau, puis reprises et incubées 10 min à 30 °C dans du tampon 0,5 M β-mercaptoéthanol, 0,1 M Tris-HCl, pH 9,3. Les cellules sont ensuite lavées avec du tampon 0,5 M KCl, 10 mM TrisHCl, pH 7, puis resuspendues et incubées pendant 20 à 40 min à 30 °C dans du tampon 1,35 M sorbitol, 1 mM EGTA, 10 mM acide citrique, 30 mM phosphate de sodium pH 5,8 (10 ml par gramme de poids sec) contenant 2 mg/ml de zymoliase 20 000 U (ICN). A ce stade les cellules sont appelées des protoplastes, c'est à dire des cellules dont la paroi a été digérée. Les protoplastes sont récoltés par centrifugation (5 min à 750 x g à 4°C) puis lavés avec du tampon 0,75 M sorbitol, 0,4 M mannitol, 0,1 % (p/v) BSA, 10 mM Tris-maléate, pH 6,8. Ils sont ensuite lysés avec un tampon 0,6 M mannitol, 2 mM EGTA, 10 mM Tris-maléate pH 6,8. Les mitochondries du lysat résultant sont ensuite récupérées par centrifugation différentielle. Une première centrifugation à basse vitesse (10 min à 750 x g) permet de se débarrasser des noyaux et débris de parois cellulaires, tandis que les mitochondries restent dans la fraction surnageante. Celle-ci est prélevée puis centrifugée à haute vitesse (10 min à 12000 x g) pour la récolte des mitochondries. Le culot de mitochondries est repris dans du tampon 0,6 M sorbitol, 2 mM EGTA, 10 mM Tris-maléate pH 6,8.

### b) Mesure de la vitesse de consommation d'oxygène des mitochondries

La vitesse de consommation d'oxygène des mitochondries est mesurée par polarographie avec une électrode de Clark (GILSON) dans du tampon 0,6 M mannitol/ 0,3 mM EGTA/ 10 mM Tris-maléate pH 6,8, contenant 3 mM de Pi/Tris pH 6,8, selon la procédure décrite par Rigoulet, M. et Guerin, M., FEBS Lett., 1979, 102, 18-22.

### c) Analyse du potentiel électrique mitochondrial

Les variations de potentiel mitochondrial ont été analysées avec de la rhodamine 123 (SIGMA) dans du tampon 0,6 M mannitol, 0,3 mM EGTA, 10 mM Tris-maléate pH 6,8, contenant 3 mM de Pi/Tris pH 6,8, avec un fluorimètre SFM25 (KONTRON), selon la procédure décrite par Emaus et al., Biochem. Biophys. Acta, 1986, 850, 436-448.

### d) Mesure de l'activité d'hydrolyse et de synthèse d'ATP mitochondriale

L'activité d'hydrolyse d'ATP mitochondriale a été mesurée dans un tampon 0,2 M KCl, 3 mM MgCl₂, 10 mM Tris-HCl pH 8,4, en présence ou en absence d'oligomycine, selon la procédure décrite par Somlo M., Eur. J. Biochem., 1968, 5, 276-284. L'activité de synthèse d'ATP mitochondriale a été mesurée, selon le protocole décrit dans Schwimmer et al., J. Biol. Chem., 2005, 280, 30751-30759. De manière plus précise, cette activité de synthèse d'ATP mitochondriale est mesurée dans du tampon 0,6 M mannitol, 0,3 mM EGTA, 10 mM Tris-maléate pH 6,8, contenant 3 mM de Pi/Tris pH 6,8, avec du NADH (4 mM) comme substrat respiratoire et en présence d'ADP (1 mM). Après l'ajout de l'ADP, une fraction du milieu réactionnel est prélevé toutes les 15 secondes (pendant 1 à 2 min) et immédiatement mélangée avec de l'acide perchlorique (7 %) et de l'EDTA (25 mM). Les échantillons sont centrifugés (5 min à 15000g) et les surnageants ajustés à pH 6 avec une solution 2N KOH, 0,3M d'acide morpholino-3-propane-sulfonique. L'ATP des échantillons est mesuré par bioluminescence, à l'aide du kit fourni par BIOTHEMA.

### e) Electrophorèse en gel non-dénaturant (technique BN-PAGE)

Des mitochondries de la souche sauvage (MR6) et du mutant t8993g ont été isolées puis solubilisées avec de la digitonine (0,75 % à 2 % p/v). Après centrifugation, les complexes ont été séparés par electrophorèse en gel non-dénaturant (technique BN-PAGE), selon la procédure décrite par Paumard et al., EMBO, J., 2002, 21, 221-230, puis les gels ont été colorés au bleu de Coomassie.

### 2) Résultats

### a) Effet des mutations sur la croissance respiratoire de la levure

La souche sauvage (MR6), le délétant Δatp6::ARG8m (MR10) et les mutants t8993g (MR14), t8993c (RKY20-1), t9176g (RKY25-4), t9176c (RKY38-1) et t8851c (RKY39-1) ont été cultivés pendant une nuit dans un milieu glucosé (YPGA). Les cultures ont été diluées de façon sériée, et une goutte de chaque dilution a été déposée sur du milieu YPGA et un milieu contenant du glycérol comme source de carbone (N3). Les boîtes ont en suite été incubées à 28°C ou 37 °C et photographiées après 4 et 7 jours d'incubation ; la boîte YPGA a été photographiée après 4 jours d'incubation à 28 °C (Figure 6, Tableau I).

**Tableau I: Effet des mutations du gène ATP6 sur la croissance respiratoire de la levure.**

| Souche de levure | Nucléotide changé chez l'homme | Changement d'acide aminé chez l'homme | Changement de codon correspondant chez la levure | Changement d'acide aminé correspondant chez la levure | Croissance respiratoire à 28°C |
|---|---|---|---|---|---|
| Sauvage (MR6) | | | | - | +++ |
| MR14 | t8993g | L156>R | tta₁₈₃>aga | L183>R | -/+ |
| RKY20-1 | t8993c | L156>P | tta₁₈₃>cca | L183>P | +++ |
| RKY25-4 | t9176g | L217>R | tta₂₄₇>aga | L247>R | - |
| RKY38-1 | t9176c | L217>P | tta₂₄₇>cca | L247>P | +++ |
| RKY39-1 | t8851c | W109>R | tga₁₃₆>aga | W136>R | -/+ |

La souche mutante MR14 portant un équivalent (tta₁₈₃>aga de la mutation t8993g présente un fort défaut de croissance sur des milieux contenant une source de carbone non fermentescible (glycérol), tant à 28 °C qu'à 37 °C; on note seulement une très légère pousse sur glycérol après sept jours d'incubation alors que la croissance de la souche sauvage est déjà complète après à peine trois jours (Figure 6). En revanche, MR14 croît normalement par la voie fermentaire (glucose) (Figure 6). Le défaut de croissance respiratoire de MR14 est totalement complémenté par croisement avec SDC30, une souche (ρ⁻ synthétique) ne contenant que le gène *ATP6* dans ses mitochondries (Figure 5). Ceci permet de conclure que la mutation t8993g et elle seule est bien responsable du défaut de croissance respiratoire observé.

De même que la mutation t8993g, les mutations t9176g et t8851c affectent très sévèrement la croissance respiratoire de la levure (Tableau I).

En revanche les mutations t8993c et t9176c n'ont pas d'incidence marquée sur la croissance respiratoire de la levure (Tableau I).

### b) Influence de la mutation t8993g sur l'activité respiratoire

Les mitochondries ont été isolées de la souche MR14 portant la mutation t8993g et de la souche parentale MR6 (les deux souches ne diffèrent génétiquement que par la mutation t8993g). La vitesse de consommation d'oxygène (respiration) des mitochondries a ensuite été mesurée par oxygraphie. Brièvement, du NADH est ajouté à la suspension mitochondriale, comme substrat respiratoire. De l'ADP est ensuite ajouté pour établir l'état 3 (état phosphorylant). Après cet ajout, on note normalement une augmentation de la vitesse de respiration suite à une consommation du gradient électrochimique de protons par l'ATP synthase qui phosphoryle l'ADP ajouté. La chaîne respiratoire fonctionne alors plus rapidement pour compenser cette consommation de protons. Lorsque tout l'ADP ajouté a été phosphorylé, la vitesse de respiration diminue pour revenir à l'état basal (état 4 non phosphorylant). Le rapport des vitesses de respiration à l'état 3 et à l'état 4 (que l'on appelle contrôle respiratoire, RCR) avait pour la souche sauvage MR6 une valeur de 2,4 typique pour des mitochondries sauvages (Figure 7). Dans le cas du mutant MR14, la vitesse de la respiration à l'état 4 est près de trois fois plus faible comparativement à la vitesse de respiration à l'état 4 mesurée pour MR6 (81 contre 298 O.min⁻¹.mg⁻¹). D'autre part on note qu'un ajout d'ADP a peu d'influence sur la vitesse de respiration. En présence d'agent découplant (CCCP: carbonyl cyanide m-chlorophenylhydrazone) qui est un ionophore à protons laissant passer librement les protons à travers la membrane, la vitesse de respiration est maximale (Vmax). La vitesse de respiration est stimulée par un facteur 4 (comparativement à l'état 4) chez le sauvage. On note également une stimulation de la respiration chez le mutant dans des proportions similaires.

### c) Mesure de la synthèse d'ATP par le complexe ATP synthase

L'activité de synthèse d'ATP par le complexe ATP synthase a été mesurée en présence d'un excès d'ADP, c'est à dire à l'état 3 (état phosphorylant). La mesure est faite en présence et en l'absence d'oligomycine, un inhibiteur spécifique du canal à protons de l'ATP synthase, de manière à déterminer la part de la vitesse de synthèse d'ATP mesurée qui est due à l'activité du complexe ATP synthase (d'autres réactions intramitochondriales sont capables de synthétiser de l'ATP). Pour la souche parentale, la valeur obtenue était de 737 ± 45 nmol.min⁻¹.mg⁻¹ (Figure 7). Dans ces conditions, la vitesse de synthèse d'ATP était beaucoup plus faible chez le mutant, 59 ±7 nmol.min⁻¹.mg⁻¹ (Figure 7).

### d) Analyse du potentiel électrique mitochondrial.

Le potentiel électrique mitochondrial a été analysé par une technique utilisant de la Rhodamine 123, une sonde fluorescente sensible à ce potentiel (Figure 8). Une augmentation du potentiel mitochondrial entraîne une entrée de Rhodamine 123 dans les mitochondries qui s'accompagne d'une diminution de fluorescence, du fait que la Rhodamine 123 est piégée dans les mitochondries. Ainsi, on peut détecter des variations du potentiel mitochondrial en mesurant des variations de fluorescence. De manière plus précise, de l'éthanol est ajouté aux mitochondries pour faire fonctionner la chaîne respiratoire, ce qui provoque l'énergisation de la membrane interne et donc une diminution de fluorescence (Figure 8). L'ADP est ensuite ajouté pour induire le fonctionnement de l'ATP synthase. Celle-ci va phosphoryler l'ADP et pour cela elle consomme le gradient de protons ce qui se traduit par une augmentation de la fluorescence. Au fur et à mesure de la phosphorylation de l'ADP ajouté, le potentiel augmente à nouveau (de moins en moins de protons sont consommés par l'ATP synthase) et on revient à la valeur du potentiel initial lorsque tout l'ADP ajouté a été phosphorylé. Pour le mutant MR14, l'ajout d'ADP n'entraîne qu'une très faible diminution de potentiel et on revient très lentement à la valeur du potentiel initial. Cette observation indique un défaut dans le fonctionnement de l'ATP synthase. Pour mieux définir cette déficience, l'énergisation de la membrane mitochondriale par l'ATP a été analysée. Dans ce cas, l'ATP synthase est étudiée en mode réverse, c'est à dire lorsqu'elle hydrolyse de l'ATP. Normalement, en hydrolysant de l'ATP, l'ATP synthase expulse des protons à l'extérieur de la mitochondrie. Dans ce mode de fonctionnement, l'ATP synthase énergise donc positivement l'extérieur de la membrane mitochondriale interne. Chez le sauvage, immédiatement après l'ajout d'ATP (en présence de KCN pour inhiber la chaîne respiratoire), on note l'établissement d'un potentiel mitochondrial important et stable. Un ajout subséquent d'oligomycine (un inhibiteur du canal à protons de l'ATP synthase) entraîne comme attendu la perte de ce potentiel, ce qui montre que celui-ci est bien lié à l'activité de l'ATP synthase. Chez le mutant MR14 on note également, immédiatement après l'ajout d'ATP, une diminution de fluorescence mais plus faible. De plus, progressivement et sans ajout d'oligomycine, on revient à la valeur initiale de fluorescence. L'ATP synthase chez le mutant n'est donc pas capable d'énergiser correctement la membrane mitochondriale interne. Ces observations montrent que la mutation t8993g est responsable d'un défaut majeur dans le fonctionnement de l'ATP synthase. Une étude similaire réalisée avec les autres mutants indique que la mutation t9176g abolit le fonctionnement de l'ATP synthase, alors que la mutation t8993c affecte bien le fonctionnement de l'ATP synthase mais de manière beaucoup moins importante que la mutation t8993g.

### e) La mutation t8993g n'influence pas l'assemblage et la stabilité de l'ATP synthase

L'influence de la mutation t8993g sur l'assemblage ou la stabilité du complexe ATP synthase a été étudiée. Des mitochondries des souches sauvage (MR6) et mutante (MR14) ont été traitées avec de la digitonine à des concentrations qui permettent de préserver les interactions au sein des complexes multiprotéiques. Les mitochondries ont ensuite été analysées par électrophorèse en gel non-dénaturant (technique BN-PAGE). Les résultats montrent que le complexe ATP synthase est parfaitement assemblé et s'accumule normalement chez le mutant (Figure 9).

### Exemple 3: Criblage de molécules actives contre les pathologies mitochondriales impliquant un déficit de phosphorylation oxydative comme le syndrome NARP.

Les mutants t8993g, t9176g et t8851c croissent très lentement à partir d'une source de carbone non fermentescible en raison d'un dysfonctionnement de l'ATP synthase. Ces mutants de levure sont donc utilisés pour identifier des molécules capables de corriger les effets de la mutation en restaurant, soit le fonctionnement de l'ATP synthase, soit la production d'ATP par les mitochondries. Les molécules capables de restaurer le fonctionnement de l'ATP synthase sont potentiellement utilisables comme médicament pour le traitement du syndrome NARP. Les molécules capables de restaurer la production d'ATP par les mitochondries sont potentiellement utilisables comme médicament pour le traitement des pathologies mitochondriales impliquant un déficit de production d'ATP par la voie de la phosphorylation oxydative ; il s'agit de pathologies liées à un dysfonctionnement du système énergétique mitochondrial, telles que notamment le syndrome NARP, lié à un dysfonctionnement de l'ATP synthase et les syndromes LHON (Leber's Hereditary Ootic Neuropathy), MILS (Maternally Inherited Leigh Syndrome), MERRF (Myoclonic Epilepsy with Ragged-Red Fibers) et HSP (Hereditary Spastic Paraplegia), liés à un dysfonctionnement des complexes respiratoires.

Le principe du test de criblage est décrit dans Bach et al., Nature Biotechnology, 2003, 21, 1075-1081. De manière plus précise, le criblage est effectué selon les étapes suivantes (Figure 10) : Etape 1: culture du mutant en milieu glucosé. Etape 2 : les cellules mutantes sont étalées en tapis à la surface d'un milieu gélosé contenant une source de carbone non-fermentescible telle que le glycérol. Etape 3: des filtres contenant chacun une quantité définie de l'une des molécules à tester sont disposés sur la boîte de pétri, les molécules diffusent dans le milieu et établissent un gradient de concentration autour des filtres. Etape 4 : les boîtes sont mises en incubation. Dans ces conditions, on voit apparaître un halo de croissance autour des filtres contenant une substance capable de contrecarrer les effets de la mutation (drogue C5).

### Exemple 4: Mise en évidence de suppresseurs intragéniques de la mutation t8993g

Des suppresseurs intragéniques de la mutation t8993g, c'est-à-dire des mutations dans le gène *ATP6* qui permettent de restaurer un fonctionnement suffisant de l'ATP synthase, ont été recherchées. Pour cela, des cellules du mutant t8993g de levure ont été étalées en tapis dense à la surface d'un milieu nutritif contenant une source de carbone non fermentescible (glycérol, milieu N3), c'est-à-dire des conditions qui ne permettent pas la croissance du mutant t8993g. On voit apparaître après quelques jours d'incubation des clones révertants ayant récupéré une capacité respiratoire suffisante (Figure 11). Les gènes *ATP6* de ces révertants ont été amplifiés par PCR et séquencés. Cette analyse a révélé différents suppresseurs intragéniques (Figure 11). Certains étaient au niveau du codon modifié par la mutation t8993g. Ces suppresseurs, dits 'premier-site' conduisent au remplacement de l'arginine 183 par un autre acide aminé que la leucine présente dans la séquence sauvage d'Atp6p, à savoir la lysine, l'isoleucine, ou la sérine. Ces résultats montrent que la présence d'une leucine dans cette position de la protéine n'est pas absolument indispensable au fonctionnement de l'ATP synthase. Chez d'autres révertants, la mutation suppresseur était localisée dans un autre codon que celui modifié par la mutation T8993G. Ces suppresseurs dits 'second-site' conduisent au remplacement du résidu arginine 179 par la sérine, du résidu alanine 180 par la proline ou la glycine, ou du résidu isoleucine 226 par la sérine (Figure 11). Il apparaît donc que des changements discrets dans la protéine Atp6p permettent de compenser la présence d'une arginine en position 183.

Ces résultats indiquent que des petites molécules susceptibles de se lier spécifiquement à l'ATP synthase, dans le voisinage de la région d'Atp6p modifiée par la mutation t8993g, pourraient restaurer le fonctionnement de l'ATP synthase en induisant un changement discret de conformation permettant de relâcher la contrainte causée par la mutation t8993g. Ces molécules représentent une des cibles pharmacologiques potentielles pouvant être utilisées pour le traitement du syndrome NARP. De telles molécules peuvent être sélectionnées par le test de criblage décrit à l'exemple 3, à l'aide des mutants t8993g, t9176g et t8851c de levure qui croissent très lentement à partir d'une source de carbone non fermentescible en raison d'un dysfonctionnement de l'ATP synthase. La restauration du fonctionnement de l'ATP synthase, par l'une de ces molécules se traduit par une restauration de la croissance des mutants, facilement détectable en milieu gélosé.

### Exemple 5: Mise en évidence de suppresseurs métaboliques de la mutation t8993g

Un mécanisme de correction (par suppression multicopie) d'une mutation nucléaire entraînant un défaut dans l'assemblage de l'ATP synthase a été mis en évidence (Schwimmer et al., J. Biol. Chem., 2005, 280, 30751-30759), Cette mutation (désignée par *Δfmc*) est un allèle nul (délétion complète) du gène nucléaire *FMCI qui* code pour une protéine (Fmc1p) de la matrice mitochondriale, indispensable à l'assemblage du secteur F₁ de l'ATP synthase (Lefebvre-Legendre et al., J. Biol. Chem., 2001; 276, 6789-6795). Le mutant *Δfmcl* présente un fort défaut de croissance respiratoire à des températures proches de 37°C ; à 28°C il croît normalement, indiquant que Fmc1p est nécessaire dans une étape de l'assemblage de l'ATP synthase sensible à la chaleur. La croissance respiratoire du mutant Δfmcl est restaurée par la surexpression d'Odclp dans les cellules, par un accroissement du nombre de copies de son gène, (Schwimmer et al., 2005, précité). La protéine Odclp est un transporteur de dicarboxylates (α-cétoglurate et α-cétoadipate) localisé dans la membrane mitochondriale interne (Palmieri et al., J. Biol., Chem., 2001, 276, 1916-1922). Dans la souche *Δfmc1* surexprimant Odc1p, le défaut en ATP synthase dû à l'inactivation de *FMC1* est toujours présent. L'accroissement du flux de dicarboxylates entre le cytosol et la matrice mitochondriale consécutif à la surexpression d'Odclp permet une plus grande production intramitochondriale d'ATP par des phosphorylations d'ADP couplées à la réaction du cycle de Krebs de décarboxylation oxydative de l'α-cétoglutarate ('phosphorylation au niveau du substrat', figure 12). Il s'agit donc d'un mécanisme de suppression métabolique, qui agit en court-circuitant le défaut en ATP synthase dans le mutant Δfmcl.

L'effet de la surexpression d'Odcp1 sur le défaut de fonctionnement de l'ATP-synthase causé par la mutation t8893g a été analysé, *in vitro* et *in vivo.*

Les souches sauvage (MR6), mutante t8993g (MR14), et mutante t8993g surexprimant Odc1p (MR14/ODC1) ont été cultivées pendant une nuit dans un milieu glucosé (YPGA). Les cultures ont été diluées de façon sériée, et une goutte de chaque dilution a été déposée sur du milieu YPGA et un milieu contenant du glycérol comme source de carbone (N3). Les boîtes ont ensuite été incubées à 36°C et ensuite photographiées après 7 jours d'incubation (Figure 12). *In vivo,* l'amélioration de la croissance respiratoire est nettement moins efficace que dans le cas du mutant Δfmc1 et n'est vraiment significative qu'à des températures proches de 37°C (Figure 12). L'analyse des protéines extraites des souches MR6, MR14 et MR14/ODC1, par Western- blot avec des anticorps dirigés contre Odclp, confirme qu'Odcp1 est surexprimée dans la souche MR14/ODC1 (Figure 12).

Les mitochondries ont été isolées des souches sauvage (MR6), mutante t8993g (MR14), et mutante t8993g surexprimant Odclp (NARP 2m ODC) cultivées en YPGAL à 37 °C, en présence des substances suivantes : 0,15 mg/ml de protéines, 4 mM NADH (état 4), 400 µM ADP (état 3), 6 µg/ml d'oligomycine, 3 µM CCCP, 15 mM Ascorbate (Asc), 1,4 mM TMPD, α-cétoglutarate 5 mM (α-KG) (Tableaux II & III).

*In vitro* les mitochondries isolées de la souche mutante t8993g surexprimant Odc1p possèdent une activité de synthèse d'ATP proche de celle mesurée avec les mitochondries de la souche sauvage correspondante, lorsque l'on utilise l'α-cétoglutarate comme substrat respiratoire (Tableau III).

**Tableau II: Activités de la chaîne respiratoire et du complexe ATP synthase sur NADH**

| Souche | % ρ-/ρ^{0°} | Vitesse de consommation d'oxygène (nAt/min/mg) | | | | Synthèse d' ATP sur NADH (pmol/min/mg) | | |
|---|---|---|---|---|---|---|---|---|
| | | NADH | | | Asc/TMPD | | | |
| | | Etat 4 | Etat 3 | +CCCP | +CCCP | -oligo | +oligo | Comp.V |
| WT | 27±1, | 93±4,5 | 287±7 | 596±2 | 886±15 | 383±15 | 95±8 | 288±10 |
| NARP | 24±0, | 50±9 | 50±9 | 95±13 | 132±16 | 39±6 | 8±2 | 30±4 |
| NARP 2m ODC | 19±1 | 45±0,2 | 109±0,5 | 254±5 | 378±12 | 74±12 | 7±1 | 67±11 |

| | | | | | | | | |
|---|---|---|---|---|---|---|---|---|
| * Les pourcentages de petites ρ⁻/ρ° dans les cultures sont indiqués. | | | | | | | | |

**Tableau III: Activités de la chaîne respiratoire et du complexe ATP synthase sur α-cétoglutarate**

| Souche | % ρ-/ρ* | Consommation d'oxygène | | Synthèse d' ATP sur α-KG (pmol/min/mg) | | |
|---|---|---|---|---|---|---|
| | | α-KG | | | | |
| | | -ADP | +ADP | -oligo | +oligo | Comp.V |
| WT | 27±1, | 22 | 125±1,5 | 200±3 | 71±2 | 132±3 |
| NARP | 24±0, | 25±2 | 28±5 | 43±5 | 17±1 | 27±3 |
| NARP 2m ODC | 19±1 | 20±0,4 | 91±1 | 166±1 | 49±4 | 117±3 |

| | | | | | | |
|---|---|---|---|---|---|---|
| * Les pourcentages de petites ρ*/ρ° dans les cultures sont indiqués. | | | | | | |

Les résultats montrent clairement deux effets bénéfiques distincts de la surexpression d'Odc1p : (i) les complexes respiratoires, surtout le complexe IV, s'accumulent d'avantage et (ii) la fraction de la production d'ATP par des phosphorylations d'ADP au niveau du substrat s'accroît de manière significative.

Ces données montrent qu'il est possible de compenser une mutation qui inactive l'ATP synthase comme la mutation t8993g, via un mécanisme qui ne restaure pas le fonctionnement de l'ATP synthase mais agit en stimulant une autre source mitochondriale de production d'ATP, à savoir la réaction de décarboxylation oxydative de l'α-cétoglutarate qui est couplée à une phosphorylation, d'ADP. Un tel mécanisme pourrait également compenser des déficits de phosphorylation oxydative dus à des mutations affectant d'autres enzymes que l'ATP synthase, en particulier les complexes respiratoires. Ceux-ci sont souvent impliqués dans des pathologies, comme LHON (Leber's Hereditary Optic Neuropathy), MILS (Maternally Inherited Leigh syndrome), MERRF (Myoclonic Epilepsy with Ragged-Red Fibers), HSP (Hereditary Spastic Paraplegia). Ainsi, une molécule capable de court-circuiter la mutation t8993g par un mécanisme tel que celui mis en évidence ci-dessus, pourrait également agir contre d'autres pathologies associées à des mutations altérant la production d'ATP par la voie des phosphorylations oxydatives.

## Revendications

1. Cellule de levure modifiée, **caractérisée en ce que** qu'elle comprend au moins une mutation du codon tryptophane 136 (W₁₃₆), leucine 183 (L₁₈₃), ou leucine 247 (L₂₄₇) du gène mitochondrial *ATP6*.

2. Cellule selon la revendication 1, **caractérisée en ce que** la dite mutation est une substitution du codon tryptophane ou leucine par un codon arginine ou proline.

3. Cellule selon la revendication 2, **caractérisée en ce qu'**elle comprend une mutation sélectionnée dans le groupe constitué par : W136R, L183R, et L247R.

4. Cellule selon l'une quelconque des revendications 1 à 3, **caractérisée en ce qu'**elle dérive d'une souche *rho⁺* de *Saccharomyces cerevisiae.*

5. Utilisation d'une cellule selon l'une quelconque des revendications 1 à 4, pour le criblage de médicaments actifs contre les pathologies mitochondriales impliquant un déficit de production d'ATP par la voie de la phosphorylation oxydative.

6. Utilisation selon la revendication 5, **caractérisée en ce que** ladite pathologie est le syndrome NARP.

7. Procédé de criblage de médicaments actifs contre les pathologies mitochondriales impliquant un déficit de production d'ATP par la voie de la phosphorylation oxydative, **caractérisé en ce qu'**il comprend :
a) la culture d'une cellule de levure modifiée selon l'une quelconque des revendications 1 à 4, en présence d'une molécule à tester, dans un milieu contenant une source de carbone non-fermentescible, et
b) l'identification des molécules capables de restaurer la croissance desdites cellules de levure modifiées.

8. Procédé selon la revendication 7, **caractérisé en ce que** ledit milieu de culture à l'étape a), est un milieu gélosé.

9. Procédé selon la revendication 7 ou la revendication 8, **caractérisé en ce que** ladite source de carbone non-fermentescible est sélectionnée dans le groupe constitué par : le glycérol, l'éthanol et le lactate.

10. Procédé selon l'une quelconque des revendications 7 à 9, **caractérisé en ce que** ladite pathologie est le syndrome NARP.

## Claims

1. Modified yeast cell, **characterised in that** it comprises at least one mutation of the tryptophan 136 (W₁₃₆) , leucine 183 (L₁₈₃) or leucine 247 (L₂₄₇) codon of the mitochondrial *ATP6* gene.

2. Cell according to claim 1, **characterised in that** said mutation is a substitution of the tryptophan or leucine codon by an arginine or proline codon.

3. Cell according to claim 2, **characterised in that** it comprises a mutation selected from the group constituted by: W136R, L183R and L247R.

4. Cell according to any one of claims 1 to 3, **characterised in that** it is derived from an *rho*⁺ strain of *Saccharomyces cerevisiae.*

5. Use of a cell according to any one of claims 1 to 4 for screening for medicaments that are active against mitochondrial pathologies involving a deficit of ATP production by the oxidative phosphorylation pathway.

6. Use according to claim 5, **characterised in that** said pathology is the NARP syndrome.

7. Method of screening for medicaments that are active against mitochondrial pathologies involving a deficit of ATP production by the oxidative phosphorylation pathway, **characterised in that** it comprises:
a) culturing a modified yeast cell according to any one of claims 1 to 4 in the presence of a test molecule, in a medium containing a non-fermentable carbon source, and
b) identifying the molecules capable of restoring the growth of said modified yeast cells.

8. Method according to claim 7, **characterised in that** said culture medium in step a) is a gelosed medium.

9. Method according to claim 7 or claim 8, **characterised in that** said non-fermentable carbon source is selected from the group constituted by: glycerol, ethanol and lactate.

10. Method according to any one of claims 7 to 9, **characterised in that** said pathology is the NARP syndrome.

## Patentansprüche

1. Modifizierte Hefezelle, **dadurch gekennzeichnet, dass** sie wenigstens eine Mutation des Tryptophan-136 (W₁₃₆)-, Leucin-183 (L₁₈₃) - oder Leucin-247 (L₂₄₇) -Codons des mitochondrialen Gens *ATP6* umfasst.

2. Zelle nach Anspruch 1, **dadurch gekennzeichnet, dass** die Mutation ein Austausch des Tryptophan- oder Leucin-Codons durch ein Arginin- oder Prolin-Codon ist.

3. Zelle nach Anspruch 2, **dadurch gekennzeichnet, dass** sie eine Mutation umfasst, die aus der Gruppe, bestehend aus W136R, L183R und L247R, ausgewählt ist.

4. Zelle nach einem der Ansprüche 1 bis 3, **dadurch gekennzeichnet, dass** sie von einem Stamm *rho*⁺ von *Saccharomyces cerevisiae* stammt.

5. Verwendung einer Zelle nach einem der Ansprüche 1 bis 4 zur Durchmusterung von Medikamenten, die gegen mitochondriale Pathologien aktiv sind, welche ein Defizit bei der Produktion von ATP durch oxidative Phosphorylierung implizieren.

6. Verwendung nach Anspruch 5, **dadurch gekennzeichnet, dass** die Pathologie das NARP-Syndrom ist.

7. Verfahren zur Durchmusterung von Medikamenten, die gegen mitochondriale Pathologien aktiv sind, welche ein Defizit bei der Produktion von ATP durch oxidative Phosphorylierung implizieren, **dadurch gekennzeichnet, dass** es umfasst:
a) die Kultur einer modifizierten Hefezelle nach einem der Ansprüche 1 bis 4 in Gegenwart eines zu testenden Moleküls in einem Medium, das eine nicht-fermentierbare Kohlenstoffquelle enthält, und
b) die Identifizierung der Moleküle, die fähig sind, das Wachstum der modifizierten Hefezelle wiederherzustellen.

8. Verfahren nach Anspruch 7, **dadurch gekennzeichnet, dass** das Kulturmedium in Stufe a) ein Agar-Medium ist.

9. Verfahren nach Anspruch 7 oder Anspruch 8, **dadurch gekennzeichnet, dass** die nicht-fermentierbare Kohlenstoffquelle aus der Gruppe, bestehend aus Glycerin, Ethanol und Lactat, ausgewählt ist.

10. Verfahren nach einem der Ansprüche 7 bis 9, **dadurch gekennzeichnet, dass** die Pathologie das NARP-Syndrom ist.


### SEQUENCE LISTING

<110> UNIVERSITE VICTOR SEGALEN BORDEAUX 2 CENTRE NATIONAL DE LA RECHERCHE SCIENTIFIQUE
   DI RAGO, Jean-Paul
   RAK, Malgorzata
   KUCHARCZYK, Roza
   TETAUD, Emmanuel
   DUVEZIN-CAUBET, Stéphane
<120> Modélisation chez la levure des mutations du gène mitochondrial ATP6 responsables du syndrome NARP chez l'homme et ses applications pour le criblage de médicaments.
<130> F1956PCT1
<160> 6
<170> PatentIn version 3.3
<210> 1
   <211> 780
   <212> DNA
   <213> Saccharomyces cerevisiae
<400> 1
   atgtttaatt tattaaatac atatattaca tcaccattag atcaatttga gattagacta      60
   ttatttggtt tacaatcatc atttattgat ttaagttgtt taaatttaac aacattttca     120
   ttatatacta ttattgtatt attagttatt acaagtttat atctattaac taataataat     180
   aataaaatta ttggttcaag atgattaatt tcacaagaag ctatttatga tactattata     240
   aatatgctta aaggacaaat tggaggtaaa aattgaggtt tatatttccc tatgatcttt     300
   acattattta tgtttatttt tattgctaat ttaattagta tgattccata ctcatttgca     360
   ttatcagctc atttagtatt tattatctct ttaagtattg ttatttgatt aggtaatact     420
   attttaggtt tatataaaca tggttgagta ttcttctcat tattcgtacc tgctggtaca     480
   ccattaccat tagtaccttt attagttatt attgaaactt tatcttattt cgctagagct     540
   atttcattag gtttaagatt aggttctaat atcttagctg gtcatttatt aatggttatt     600
   ttagctggtt tactatttaa ttttatgtta attaatttat ttactttagt attcggtttt     660
   gtacctttag ctatgatctt agccattatg atgttagaat tcgctattgg tatcattcag     720
   ggatatgtct gggctatttt aacagcatca tatttaaaag atgcagtata cttacattaa     780
<210> 2
   <211> 259
   <212> PRT
   <213> Saccharomyces cerevisiae
<400> 2
   Met Phe Asn Leu Leu Asn Thr Tyr Ile Thr Ser Pro Leu Asp Gln Phe
   1               5                   10                  15
   Glu Ile Arg Thr Leu Phe Gly Leu Gln Ser Ser Phe Ile Asp Leu Ser
               20                  25                  30
   Cys Leu Asn Leu Thr Thr Phe Ser Leu Tyr Thr Ile Ile Val Leu Leu
           35                  40                  45
   Val Ile Thr Ser Leu Tyr Thr Leu Thr Asn Asn Asn Asn Lys Ile Ile
       50                  55                  60
   Gly Ser Arg Trp Leu Ile Ser Gln Glu Ala Ile Tyr Asp Thr Ile Met
   65                  70                  75                  80
   Asn Met Thr Lys Gly Gln Ile Gly Gly Lys Asn Trp Gly Leu Tyr Phe
                   85                  90                  95
   Pro Met Ile Phe Thr Leu Phe Met Phe Ile Phe Ile Ala Asn Leu Ile
               100                 105                 110
   Ser Met Ile Pro Tyr Ser Phe Ala Leu Ser Ala His Leu Val Phe Ile
           115                 120                 125
   Ile Ser Leu Ser Ile Val Ile Trp Leu Gly Asn Thr Ile Leu Gly Leu
       130                 135                 140
   Tyr Lys His Gly Trp Val Phe Phe Ser Leu Phe Val Pro Ala Gly Thr
   145                 150                 155                 160
   Pro Leu Pro Leu Val Pro Leu Leu Val Ile Ile Glu Thr Leu Ser Tyr
                   165                 170                 175
   Phe Ala Arg Ala Ile Ser Leu Gly Leu Arg Leu Gly Ser Asn Ile Leu
               180                 185                 190
   Ala Gly His Leu Leu Met Val Ile Leu Ala Gly Leu Thr Phe Asn Phe
           195                 200                 205
   Met Leu Ile Asn Leu Phe Thr Leu Val Phe Gly Phe Val Pro Leu Ala
       210                 215                 220
   Met Ile Leu Ala Ile Met Met Leu Glu Phe Ala Ile Gly Ile Ile Gln
   225                 230                 235                 240
   Gly Tyr Val Trp Ala Ile Leu Thr Ala Ser Tyr Leu Lys Asp Ala Val
                   245                 250                 255
   Tyr Leu His
<210> 3
   <211> 88
   <212> DNA
   <213> artificial sequence
<220>
   <223> amorce ATP6-PRO
<400> 3
   gcgggatcct ttattatagt ttaatactcc atatgtaaat tattttattt tataatttta 60
   ttttataatt taagcatata cagcttcg 88
<210> 4
   <211> 88
   <212> DNA
   <213> artificial sequence
<220>
   <223> amorce ATP6-Ter
<400> 4
   gcctagataa taagatataa ttatgattaa ttattataag ttatatagtt ttataaattt 60
   ataattatta tgacacattt agaaagaa 88
<210> 5
   <211> 19
   <212> DNA
   <213> artificial sequence
<220>
   <223> amorce ATP6-up
<400> 5
   gcggacccca aaggaggag 19
<210> 6
   <211> 28
   <212> DNA
   <213> artificial sequence
<220>
   <223> amorce ATP6-down
<400> 6
   cgggatccca gtggggaagg agtgaggt 28